# EUROPÄISCHE PATENTSCHRIFT

(11) **EP 3 851 150 B1**
(45) Veröffentlichungstag und Bekanntmachung des Hinweises auf die Patenterteilung: **13.03.2024**
(21) Anmeldenummer: 21150132.5
(22) Anmeldetag: 05.01.2021
(51) Int. Cl.: A61M 16/10, A61M 16/00, G01F 1/68, A61M 11/04, A61M 16/18

(54) **ANÄSTHESIEMITTELDOSIERVORRICHTUNG MIT EINER MESSEINHEIT**
ANAESTHETIC DOSING DEVICE WITH A MEASURING UNIT
DISPOSITIF DE DOSAGE D'UNE ANESTHÉSIQUE DOTÉ D'UNE UNITÉ DE MESURE

(30) Priorität: 17.01.2020 DE 102020000268
(43) Veröffentlichungstag der Anmeldung: 21.07.2021
(73) Patentinhaber: Drägerwerk AG & Co. KGaA, 23558 Lübeck (DE)
(72) Erfinder: STARK, Hartmut, 23558 Lübeck (DE); WRUCK, Norbert, 23558 Lübeck (DE)

(56) Entgegenhaltungen:
- EP-A1- 0 659 445
- US-A- 5 546 931
- US-A1- 2007 051 163
- US-A1- 2011 155 131
- BURTE E P ET AL: "Microsystems for measurement and dosage of volatile anesthetics and respirative gases in anesthetic equipment", MICRO ELECTRO MECHANICAL SYSTEMS, 1998. MEMS 98. PROCEEDINGS., THE ELE VENTH ANNUAL INTERNATIONAL WORKSHOP ON HEIDELBERG, GERMANY 25-29 JAN. 1998, NEW YORK, NY, USA,IEEE, US, 25. Januar 1998 (1998-01-25), Seiten 510-514, XP010270162, DOI: 10.1109/MEMSYS.1998.659810 ISBN: 978-0-7803-4412-9

## Beschreibung

Die Erfindung betrifft eine Anästhesiemitteldosiervorrichtung mit einer Messeinheit zum Bestimmen einer Anästhesiemittelkonzentration im Bereich eines Auslasses der Anästhesiemitteldosiervorrichtung. Weiterhin betrifft die Erfindung ein Verfahren zum Bestimmen einer Anästhesiemittelkonzentration im Bereich eines Auslasses einer Anästhesiemitteldosiervorrichtung.

Die Verwendung einer Anästhesiemitteldosiervorrichtung zum Anreichern eines bereitgestellten Atemgases mit einem Anästhesiemittel und zum Ausgeben eines derart angereicherten Gasgemisches an einen mit einem Patienten verbundenen Anästhesieatemkreis ist bekannt. Hierbei wird typischerweise ein erster Gaszweig mit einem Anästhesiemittelverdunster verwendet, um das zugeführte Atemgas mit dem Anästhesiemittel anzureichern. Weiterhin wird typischerweise ein zweiter Gaszweig, auch Bypass-Zweig genannt, an dem Anästhesiem ittelverdunster vorbeigeführt, um im Rahmen einer Zuführung beider Gaszweige zu einer Mischereinheit ein verdünntes mit dem Anästhesiemittel angereicherten Gasgemisch bereitzustellen.

Zur Kontrolle der Dosierung des Anästhesiemittels wird in US 5,967,141 vorgeschlagen, die Anästhesiemittelkonzentration an einem Auslass der Anästhesiemitteldosiervorrichtung zu bestimmen und die Dosierung durch eine Steuerung des Gasflusses durch die Gaszweige zu verändern, falls die bestimmte Anästhesiemittelkonzentration nicht mit einer vorbestimmten Sollkonzentration übereinstimmt.

Aus US 2011 / 0 155 131 A1 ist ein Anästhesiemittelverdampfer und ein Verfahren zu dessen Steuerung bekannt. Das Verfahren umfasst ein Messen einer Zusammensetzung eines Trägergases mit einem ersten Sensor und ein Messen einer Zusammensetzung eines kombinierten Gases mit einem zweiten Sensor und die Verwendung dieser Werte mit einem Regler zur Betätigung eines Durchflussventils für die Regulierung des Durchflusses des Anästhesiemittels in Richtung des Trägergases zur Bildung des kombinierten Gases.

Aus US 5 546 931 A ist eine Vorrichtung zur Anästhesiemitteldampfabgabe mit einem Umgebungsbezogenen Druckregler bekannt.

Aus US 2007 / 0 051 163 A1 ist ein Verfahren und eine Vorrichtung bekannt, welche es ermöglichen, einem Gassensor in einer Erfassungszone wechselnde Gasproben ohne wesentliche Änderung des Probendrucks an dem Gassensor in der Erfassungszone zuzuführen, wodurch Druckschwankungen, die unerwünschte Sensorsignale erzeugen können, abgeschwächt werden.

Aus EP 0 659 445 A1 ist System zur Abgabe von Stickstoffmonoxid an einen Patienten in einer bestimmten Konzentration bekannt.

Aufgabe der vorliegenden Erfindung ist es, eine besonders einfache Bestimmung der Anästhesiemittelkonzentration, insbesondere eine besonders robuste Bestimmung der Anästhesiemittelkonzentration im Bereich des Auslasses einer Anästhesiemitteldosiervorrichtung, bereitzustellen.

Erfindungsgemäß wird zur Lösung dieser Aufgabe eine Anästhesiemitteldosiervorrichtung mit einer Messeinheit zum Bestimmen einer Anästhesiemittelkonzentration im Bereich eines Auslasses der Anästhesiemitteldosiervorrichtung vorgeschlagen, weiterhin aufweisend einen ersten Gaszweig, einen zweiten Gaszweig, einen Anästhesiemittelverdunster und eine Mischereinheit.

Der erste Gaszweig ist mit einer Atemgaszufuhr verbindbar und ist ausgebildet, ein Atemgas durch den Anästhesiemittelverdunster zu der Mischereinheit zu führen.

Der zweite Gaszweig ist mit der Atemgaszufuhr verbindbar und ist ausgebildet, das Atemgas ebenfalls zu der Mischereinheit zu führen.

Der Anästhesiemittelverdunster ist angeordnet und ausgebildet, das Atemgas in dem ersten Gaszweig mit einem Anästhesiemittel anzureichern.

Die Mischereinheit ist angeordnet und ausgebildet, das mit dem Anästhesiemittel angereicherte Atemgas aus dem ersten Gaszweig mit dem Atemgas aus dem zweiten Gaszweig zu mischen und das dadurch entstandene Gasgemisch dem Auslass der Anästhesiemitteldosiervorrichtung bereitzustellen.

Die Messeinheit weist mindestens zwei Sensorelemente auf. Ein erstes Sensorelement der mindestens zwei Sensorelemente ist angeordnet und ausgebildet, zumindest einen ersten Kennwert einer Gaskonzentrations-abhängigen Kenngröße zwischen Mischereinheit und Auslass zu messen. Ein zweites Sensorelement der mindestens zwei Sensorelemente ist angeordnet und ausgebildet, zumindest einen zweiten Kennwert der Gaskonzentrations-abhängigen Kenngröße in dem zweiten Gaszweig oder im Bereich der Atemgaszufuhr zu messen. Dabei ist die Messeinheit weiterhin ausgebildet, abhängig von einer dem zweiten Kennwert zugeordneten Kalibrierungsinformation, insbesondere einer vorbestimmten Kalibrierungsinformation, und zumindest dem ersten Kennwert, insbesondere einer Differenz zwischen dem ersten und zweiten Kennwert, die Anästhesiemittelkonzentration zwischen Mischereinheit und Auslass zu bestimmen, insbesondere zu berechnen, und ein entsprechendes Konzentrationssignal auszugeben. Das Konzentrationssignal indiziert hierbei die bestimmte Anästhesiemittelkonzentration.

Im Rahmen der Erfindung wurde erkannt, dass ein Messen einer Gaskonzentrations-abhängigen Kenngröße an zwei Orten innerhalb der Anästhesiemitteldosiervorrichtung, nämlich für das nicht mit dem Anästhesiemittel angereicherte Atemgas und für das angereicherte Atemgas im Bereich des Auslasses, das Vorsehen einer einfach strukturierten Messeinheit, insbesondere einer Messeinheit mit unselektivem Sensorelement, erlaubt. So ist die erfindungsgemäße Messeinheit vorzugsweise nicht dazu ausgebildet, aus einem einzelnen Kennwert der Gaskonzentrations-abhängigen Kenngröße eine Anästhesiemittelkonzentration zu bestimmen. Vorzugsweise wird erst durch die Berücksichtigung beider Kennwerte unter Nutzung einer einem Kennwert zugeordneten Kalibrierungsinformation das Bestimmen der Anästhesiemittelkonzentration zwischen Mischereinheit und Auslass möglich.

Das Vorsehen eines in diesem Sinne unselektiven Sensorelementes innerhalb der Messeinheit erlaubt die Verwendung einer besonders robusten Messeinheit. Weiterhin wird hierdurch eine besonders kostengünstige Bestimmung der Anästhesiemittelkonzentration ermöglicht.

Die Bestimmung der Anästhesiemittelkonzentration direkt im Bereich des Auslasses der Anästhesiemitteldosiervorrichtung erlaubt eine präzise Vorhersage, welche Anästhesiemittelkonzentration einem Patienten bei der Verwendung der Anästhesiemitteldosiervorrichtung zugeführt wird.

Durch das Bereitstellen einer besonders kostengünstigen Bestimmung der Anästhesiemittelkonzentration kann vorteilhaft eine kostengünstige redundante Messung der Gaskonzentrations-abhängigen Kenngröße und/oder Bestimmung der Anästhesiemittelkonzentration bereitgestellt werden. Diese redundante Messung erlaubt eine automatisierte gegenseitige Überwachung von Komponenten der Anästhesiemitteldosiervorrichtung. Hierdurch kann insbesondere das Risiko einer nicht erkannten oder spät erkannten Fehlfunktion der Messeinheit reduziert werden.

Vorteilhaft können für die erfindungsgemäße Anästhesiemitteldosiervorrichtung verschiedene bekannte volatile Anästhesiemittel, wie beispielsweise Isofluran, Sevofluran und Desfluran, verwendet werden. Dies erfordert lediglich das erfindungsgemäße Berücksichtigen einer entsprechenden Kalibrierungsinformation betreffend das entsprechende volatile Anästhesiemittel.

Das Messen zwischen Mischereinheit und Auslass schließt erfindungsgemäß das Messen direkt am Auslass oder direkt an der Mischereinheit mit ein.

Die Gaskonzentrations-abhängige Kenngröße kann beispielsweise eine Temperatur des Gases im Rahmen einer Wärmeleitfähigkeitsmessung, eine Dichte des Gases und/oder eine spektrale Eigenschaft des Gases umfassen. Die Struktur des mindestens einen Sensorelementes der Messeinheit ist abhängig davon, welche Gaskonzentrations-abhängige Kenngröße in dem Atemgas gemessen wird.

Erfindungsgemäß wird die dem zweiten Kennwert zugeordnete Kalibrierungsinformation bestimmt, um dadurch einen Einfluss des Anästhesiemittels auf die Gaskonzentrations-abhängige Kenngröße zu erfassen. So ist die Auswahl der Kalibrierungsinformation erfindungsgemäß nicht abhängig von der Anästhesiemittelkonzentration, sondern nur von dem zweiten Kennwert, also dem im zweiten Gaszweig ohne Anästhesiemittel gemessenen Kennwert. Anhand dieser Kalibierungsinformation wird erfindungsgemäß abhängig von dem ersten Kennwert, also abhängig von einem im mit Anästhesiemittel beladenen Gas gemessenen Kennwert, die Anästhesiemittelkonzentration bestimmen. Hierbei kann die Kalibrierungsinformation auch anhand einer Differenz zwischen erstem und zweitem Kennwert oder anhand einer anderen Größe, die sich aus dem ersten Kennwert oder dem ersten und zweiten Kennwert ergibt, verwendet werden.

Die Kalibrierungsinformation kann auch eine Mehrzahl an Kalibrierungsinformationen umfassen. Insbesondere erlaubt die Kalibrierungsinformation vorzugsweise anhand des ersten Kennwerts oder einer ermittelten Differenz zwischen dem ersten und zweiten Kennwert ein Bestimmen des in dem Anästhesiemittelverdunster verwendeten Anästhesiemittels. Vorzugsweise erlaubt die Messung des zweiten Kennwerts die Bestimmung der Zusammensetzung des als Atemgas zugeführten Frischgases, wie etwa das Verhältnis aus Sauerstoff und Lachgas oder aus Sauerstoff und Stickstoff in dem zugeführten Atemgas, insbesondere für den bevorzugten Fall, dass die Bestandteile des zugeführten Atemgases bekannt sind, beispielsweise durch eine Nutzereingabe bekannt sind. Auf der Basis eines solchen bekannten Verhältnisses kann durch die Messung des ersten Kennwerts oder die Differenz der beiden Kennwerte die Anästhesiemittelkonzentration ermittelt werden. Hierzu liegen vorzugsweise Kalibrierungsinformationen für die in dem Anästhesiemittelverdunster verwendbaren Anästhesiemittel vor.

Erfindungsgemäß liegt zumindest für ein Anästhesiemittel die Kalibrierungsinformation vor. Vorzugsweise erlaubt eine Mehrzahl an Kalibrierungsinformationen eine Verwendung der erfindungsgemäßen Anästhesiemitteldosiervorrichtung für verschiedene übliche zuzuführende Atemgase, wie etwa ein Sauerstoff-Stickstoff-Gemisch und ein Sauerstoff-Lachgas-Gemisch.

Die Kalibrierungsinformation ist vorzugsweise eine vorbestimmte Kalibrierungsinformation. Vorbestimmt ist die Kalibrierungsinformation dadurch, dass sie vor einem Betrieb hinterlegt oder zumindest vor dem aktuellen Betrieb der erfindungsgemäßen Anästhesiemitteldosiervorrichtung erzeugt wurde, beispielsweise während eines früheren Betriebs erzeugt wurde.

Die Kalibrierungsinformation kann erfindungsgemäß beispielsweise in Form eines Kennliniensatzes, einer funktionalen Abhängigkeit und/oder einer Wertetabelle vorliegen. Erfindungsgemäß liegen für verschiedene zweite Kennwerte verschiedene Kalibrierungsinformationen vor, so dass nach dem Messen des zweiten Kennwertes die zu verwendende Kalibrierungsinformation bekannt ist und diese Kalibrierungsinformation in Kombination mit zumindest dem ersten Messwert, insbesondere mit einer Differenz aus erstem und zweitem Messwert verwendet wird, um die Anästhesiemittelkonzentration zu bestimmen.

Die Zuordnung der Kalibrierungsinformation zu dem zweiten Kennwert erfolgt beispielsweise durch eine Kennlinie eine Funktion oder andere Zuordnungsvorschriften. Unter Kalibrierungsinformation wird im Rahmen der Erfindung die Gesamtheit aller verwendeten Informationen, insbesondere aller vorbestimmten Informationen verstanden, über die die beiden gemessenen Kennwerte ausgewertet werden. Neben der dem zweiten Kennwert zugeordnete Kalibrierungsinformation wird also auch die Kalibrierungsinformation verwendet, um die Zuordnung zwischen zweitem Kennwert und zugeordneter Kalibrierungsinformation bereitzustellen. Details zu der entsprechenden Auswertung sind im Rahmen der Figurenbeschreibung erläutert.

Das Konzentrationssignal kann beispielsweise an einen Nutzer der Anästhesiemitteldosiervorrichtung und/oder an eine Steuereinheit zum Steuern der Anreicherung des Atemgases mit Anästhesiemittel ausgegeben werden.

Die erfindungsgemäße Messeinheit kann aus mehreren Modulen, beispielsweise aus mehreren ein jeweiliges Sensorelement umfassenden Modulen, bestehen. Die Messeinheit kann entsprechend einteilig oder mehrteilig ausgebildet sein.

Die Messeinheit weist vorzugsweise einen Prozessor auf, der das erfindungsgemäße Bestimmen der Anästhesiemittelkonzentration ausführt. Der Prozessor kann in unmittelbarer Nähe zu dem mindestens einen Sensorelement oder alternativ beabstandet zu dem Sensorelement, beispielsweise in einem separaten Modul angeordnet sein.

Der Aufbau eines Anästhesiemittelverdunsters und einer Mischereinheit sind dem Fachmann grundsätzlich bekannt, so dass dieser im Folgenden nicht im Detail erläutert wird. Unter einem Anästhesiemittelverdunster ist im Rahmen dieser Erfindungsmeldung jede Vorrichtung zu verstehen, die ein Anästhesiemittel gasförmig einem zugeführten Gasstrom beimischt. So ist ein Anästhesiemittelverdunster im Sinne dieser Anmeldung auch eine Vorrichtung, die über ein Heizelement eine Beimischung eines Anästhesiemittels herbeiführt, wie dies etwa bei einer typischerweise als Anästhesiemittelverdampfer bezeichneten Vorrichtung der Fall ist.

Das Bestimmen eines Wertes basierend auf vorbestimmten Informationen ist im Rahmen dieser Erfindung ein Bestimmen des Wertes durch unmittelbare Verwendung der vorbestimmten Informationen und/oder durch Verwendung von Informationen, die aufgrund der vorbestimmten Informationen, beispielsweise der Kalibrierungsinformation, zur Verfügung stehen. Das Bestimmen basierend und/oder abhängig von vorbestimmten Informationen kann auch unter Verwendung weiterer Informationen bei dieser Bestimmung erfolgen.

Nachfolgend werden bevorzugte Ausführungsformen der erfindungsgemäßen Anästhesiemitteldosiervorrichtung sowie weitere erfindungsgemäße Aspekte beschrieben.

In einer besonders bevorzugten Ausführungsform ist die Messeinheit ausgebildet, abhängig von der dem zweiten Kennwert zugeordneten Kalibrierungsinformation und einer Differenz zwischen erstem und zweitem Messwert die Anästhesiemittelkonzentration zu bestimmen. Hierdurch kann besonders vorteilhaft ein Fehler in der Auswertung der beiden Kennwerte reduziert werden.

In einer besonders bevorzugten Ausführungsform der erfindungsgemäßen Anästhesiemitteldosiervorrichtung misst das mindestens eine Sensorelement die Kennwerte der Gaskonzentrations-abhängigen Kenngröße basierend auf einer Wärmeleitfähigkeitsmessung. Die Wärmeleitfähigkeitsmessung basiert dabei typischerweise auf einer Temperaturmessung zum Bestimmen der Leitfähigkeit eines auf eine Referenztemperatur aufgewärmten Messgases. Hierdurch kann besonders einfach und wenig fehleranfällig die entsprechende Gaskonzentrations-abhängige Kenngröße ermittelt werden. In einer alternativen oder ergänzenden Ausführungsform misst das mindestens eine Sensorelement die Kennwerte basierend auf einer Ultraschallmessung, auf einer Dichtemessung und/oder auf einer optischen Messung spektraler Eigenschaften des Atemgases.

Erfindungsgemäß weist die Messeinheit mindestens zwei Sensorelemente auf, wobei wie vorestehend beschrieben ein erstes Sensorelement der mindestens zwei Sensorelemente angeordnet und ausgebildet ist, zumindest den ersten Kennwert der Gaskonzentrations-abhängigen Kenngröße zwischen Mischereinheit und Auslass zu messen, und wobei ein zweites Sensorelement der mindestens zwei Sensorelemente angeordnet und ausgebildet ist, zumindest den zweiten Kennwert der Gaskonzentrations-abhängigen Kenngröße in dem zweiten Gaszweig oder im Bereich der Atemgaszufuhr zu messen. In dieser Ausführungsform können die mindestens zwei Sensorelemente zeitgleich den ersten und zweiten Kennwert der Gaskonzentrations-abhängigen Kenngröße messen und dadurch eine besonders präzise und schnelle Bestimmung der aktuell im Bereich des Auslasses vorliegenden Anästhesiemittelkonzentration ermöglichen. Insbesondere wird bei einer zeitgleichen oder nahezu zeitgleichen Messung vorteilhaft eine Druck- und/oder Konzentrationsschwankung während der Messung beider Kennwerte gleichmäßig berücksichtigt. Die mindestens zwei Sensorelemente können in einem gemeinsamen Gehäuse oder in räumlich getrennten Modulen der erfindungsgemäßen Messeinheit angeordnet sein.

Ferner erfindungsgemäß umfasst die Messeinheit mindestens zwei Schalter, wobei das erste Sensorelement der mindestens zwei Sensorelemente mit einem ersten Schalter der mindestens zwei Schalter verbunden ist und wobei das zweite Sensorelement der mindestens zwei Sensorelemente mit einem zweiten Schalter der mindestens zwei Schalter verbunden ist. Dabei sind der erste und der zweite Schalter ausgebildet, zwischen der Messung des ersten Kennwerts und der Messung des zweiten Kennwerts durch das jeweils verbundene Sensorelement umschalten zu können. In dieser Variante kann eine Fehlfunktion eines Sensorelements durch die ermöglichte redundante Messung der beiden Kennwerte besonders schnell erkannt werden. Insbesondere kleine Messfehler können durch einen Vergleich des jeweiligen über das erste Sensorelement und über das zweite Sensorelement bestimmten Kennwerts schnell erkannt werden. Um eine redundante Messung der beiden Kennwerte zu ermöglichen, wird in dieser Variante vorzugsweise wiederholt die Schalterstellung der beiden Schalter verändert. Das Umschalten der Schalter kann manuell oder automatisiert ausgelöst werden. Ein manuelles Auslösen des Umschaltens erfolgt beispielsweise über eine Benutzerschnittstelle der Anästhesiemitteldosiervorrichtung. Ein automatisiertes Umschalten erfolgt beispielsweise durch einen Prozessor der Messeinheit oder über eine separate Steuereinheit der Anästhesiemitteldosiervorrichtung. Vorzugsweise wird in regelmäßigen zeitlichen Abständen automatisiert die Schalterstellung der beiden Schalter verändert, um eine Fehlfunktion eines Sensorelement ohne manuelle Unterstützung schnell zu erkennen.

In einem besonders vorteilhaften Beispiel der vorhergehenden Variante sind der erste Schalter und der zweite Schalter miteinander derart gekoppelt, insbesondere mechanisch gekoppelt, dass ein Wechsel einer ersten Schalterstellung des ersten Schalters auch einen Wechsel einer zweiten Schalterstellung des zweiten Schalters bewirkt. In diesem Beispiel wird vermieden, dass beide Sensorelemente zeitgleich denselben Kennwert messen. So wird in diesem Beispiel sichergestellt, dass beide Kennwerte der Gaskonzentrations-abhängigen Kenngröße stets zeitgleich oder im Wesentlichen zeitgleich bestimmt werden.

In einer besonders bevorzugten Ausführungsform erfolgt die Messung des ersten Kennwerts und die Messung des zweiten Kennwerts durch die Messeinheit im Wesentlichen zeitgleich. Hierdurch kann stets der aktuelle erste und der aktuelle zweite Kennwert ermittelt werden und mithin über die Kalibrierungsinformation besonders präzise die Anästhesiemittelkonzentration zwischen Mischereinheit und Auslass bestimmt werden. Insbesondere können Fehler bei dieser Bestimmung aufgrund von Druck- und/oder Konzentrationsschwankungen vermieden werden.

In einer weiteren vorteilhaften Ausführungsform umfasst die Kalibrierungsinformation eine Mehrzahl von Atemgaszusammensetzungs-abhängigen Kalibrierungskurven, insbesondere mindestens eine jeweilige Atemgaszusammensetzungs-abhängige Kalibrierungskurve für ein Sauerstoff-Stickstoff-Gemisch und für ein Sauerstoff-Lachgas-Gemisch. In dieser Ausführungsform kann vorteilhaft zwischen einer Mehrzahl an verschiedenen zuzuführenden Atemgasen für die Anästhesiemitteldosiervorrichtung ausgewählt werden, ohne dass dies eine gesonderte Kalibrierung oder Anpassung der Vorrichtung erfordert. Vorzugsweise ist die Anästhesiemitteldosiervorrichtung, insbesondere die Messeinheit der Anästhesiemitteldosiervorrichtung, dazu ausgebildet, automatisiert anhand der beiden gemessenen Kennwerte und der Kalibrierungsinformation das verwendete Atemgas zu ermitteln. Ein Ausführungsbeispiel für eine derartige Auswertung des verwendeten Atemgases ist im Rahmen der Figurenbeschreibung erläutert. Alternativ oder ergänzend umfasst die Kalibrierungsinformation eine jeweilige Atemgaszusammensetzungs-abhängige Kalibrierungskurve für ein Sauerstoff-Argon-Lachgas-Gemisch und/oder für ein Sauerstoff-Xenon-Gemisch.

In einer weiteren Ausführungsform weist die Messeinheit eine Anzahl von Messgasführungskanälen auf, in der die Messung des ersten und zweiten Kennwerts erfolgt. Vorzugsweise ist diese Anzahl von Messgasführungskanälen in die Atemgasführung der Anästhesiemitteldosiervorrichtung derart pneumatisch eingebunden, dass temporäre Druckschwankungen innerhalb des ersten und/oder zweiten Gaszweigs auch in entsprechender Weise innerhalb der Anzahl von Messgasführungskanälen zumindest teilweise vorliegen. Hierdurch kann sichergestellt werden, dass sich temporäre Druckschwankungen nicht auf die Genauigkeit der Messung der beiden Kennwerte auswirken. Vorzugsweise weisen die Messgasführungskanälen eine geringere Querschnittsfläche auf, als die beiden Gaszweige, da lediglich ein geringes Messgasvolumen für die Messung der beiden Kennwerte notwendig ist. Durch die geringere Querschnittsfläche wird die Führung des Atemgases zu dem Auslass durch die Messung der beiden Kennwerte wenig oder im Wesentlichen nicht beeinflusst. Der Einfluss der Messung auf das Atemgasvolumen ist vorzugsweise durch die kleine Querschnittsfläche eines jeweiligen Messgasführungskanals so gering, dass er das am Auslass bereitgestellte Atemgasvolumen nicht in einer für den Nutzer der Anästhesiemitteldosiervorrichtung wahrnehmbaren Weise beeinträchtigt. Besonders vorteilhaft ist das Bereitstellen des ersten und zweiten Sensorelements in einer Variante dieser Ausführungsform, da im Rahmen des gleichzeitigen Messens der beiden Kennwerte das gleiche Atemgasvolumen, das über einen ersten Messgasführungskanal zwischen Mischereinheit und Auslass dem Atemgas zum Messen eines Kennwerts entnommen wurde, über einen zweiten Messgasführungskanal zum Messen des weiteren Kennwerts wieder zugeführt wird. Hierdurch kann der Einfluss der Messung auf das am Auslass bereitgestellte Atemgasvolumen sehr gering gehalten werden.

In einer weiteren Ausführungsform weist die erfindungsgemäße Anästhesiemitteldosiervorrichtung weiterhin eine Steuereinheit auf, die ausgebildet ist, das ausgegebene Konzentrationssignal zu empfangen und basierend auf einem Vergleich zwischen einer vorbestimmten Soll-Anästhesiemittelkonzentration und der bestimmten Anästhesiemittelkonzentration die Anreicherung des Atemgases mit Anästhesiemittel in dem ersten Gaszweig zu steuern. Die Eingabe der vorbestimmten Soll-Anästhesiemittelkonzentration kann beispielsweise über eine Benutzerschnittstelle der Anästhesiemitteldosiervorrichtung erfolgen. Das Steuern der Anreicherung des Atemgases kann beispielsweise über eine Steuerung des Atemgasvolumens innerhalb des ersten Gaszweigs, über eine Steuerung des Anästhesiemittelvolumens innerhalb des Anästhesiemittelverdunsters, über eine Steuerung der Temperatur innerhalb des Anästhesiemittelverdunsters oder über eine Steuerung des Druckes innerhalb des Anästhesiemittelverdunsters oder dergleichen erfolgen. Derartige Verfahren zur Steuerung der Anästhesiemittelkonzentration sind bekannt und werden daher im Folgenden nicht im Detail geschildert.

In einer Variante der vorhergehenden Ausführungsform ist die Steuereinheit weiterhin ausgebildet, basierend auf der Steuerung des Atemgasvolumens innerhalb des ersten Gaszweigs ein Laufzeitkorrektursignal zu bestimmen und an die Messeinheit auszugeben. Dabei ist die Messeinheit weiterhin ausgebildet, das Laufzeitkorrektursignal zu empfangen und basierend auf dem Laufzeitkorrektursignal die Anästhesiemittelkonzentration zu bestimmen. Vorzugsweise zeigt das Laufzeitkorrektursignal einen Unterschied in der aktuellen Laufzeit für Gas in dem ersten und zweiten Gaszweig an. In dieser Ausführungsform kann vorteilhaft ein Unterschied in der Zusammensetzung des zugeführten Atemgases während der gleichzeitigen Messung der beiden Kennwerte berücksichtigt werden. Beispielsweise können die Kennwerte von der Messeinheit über einen vorbestimmten Zeitraum gespeichert und im Falle einer Zusammensetzungsveränderung nach dem vorbestimmten Zeitraum korrigiert werden, wobei erst nach dem vorbestimmten Zeitraum, vorzugsweise nach mindestens 2 Sekunden, insbesondere nach mindestens 5 Sekunden, besonders bevorzugt nach etwa 10 Sekunden, die Anästhesiemittekonzentration bestimmt und an die Steuereinheit ausgegeben wird. In einer alternativen nicht dargestellten Ausführungsform ist die Steuereinheit dazu ausgebildet, die beschriebene Laufzeitkorrektur mit dem empfangenen Konzentrationssignal der Messeinheit zu verrechnen, wobei das Konzentrationssignal vorzugsweise eine Anzahl von gemessenen ersten und zweiten Kennwerten indiziert, die ein Verrechnen der Kennwerte mit der Laufzeitkorrektur zum Bestimmen der Anästhesiemittelkonzentration ermöglichen.

In einem Beispiel der vorhergehenden Variante der vorhergehenden Ausführungsform ist zur Verbesserung der Berücksichtigung der Laufzeitkorrektur eine Messung eines dritten Kennwerts der Kenngröße in dem ersten Gaszweig in Flussrichtung des Atemgases hinter dem Anästhesiemittelverdunster vorgesehen. Der dritte Kennwert zeigt eine Veränderung der Anästhesiemittelkonzentration aufgrund der Laufzeit des zugeführten Atemgases schneller an, als der erste Kennwert im Bereich des Auslasses. So kann beispielsweise durch den ersten und den dritten Kennwert auf eine Flussgeschwindigkeit innerhalb des ersten Gaszweigs geschlossen werden. Weiterhin kann durch die weitere Messung, insbesondere bei Verwendung eines weiteren Sensors, ein Fehler der Messeinheit oder einer einzelnen Komponente der Messeinheit besonders schnell erkannt werden.

In einer besonders bevorzugten Variante der vorhergehenden Ausführungsform ist die Steuereinheit weiterhin ausgebildet, ein Abschalten der Anästhesiemitteldosiervorrichtung auszulösen, falls eine Differenz zwischen bestimmter Anästhesiemittelkonzentration und vorbestimmter Soll-Anästhesiemittelmittelkonzentration einen vorbestimmten Alarmschwellwert übersteigt. Ein derartiges Abschalten kann beispielsweise über ein Schließen eines zusätzlichen Alarmventils erfolgen, welches beispielsweise zwischen Mischer und Auslass oder im ersten Gaszweig in Gasflussrichtung hinter dem Anästhesiemittelverdunster angeordnet ist. In dieser Variante kann über das Abschalten sichergestellt werden, dass nicht eine deutlich zu hohe Anästhesiemittelkonzentration dem Anästhesieatemkreis zugeführt wird. Auch das Verabreichen einer dauerhaft zu geringen Anästhesiemittelkonzentration kann durch einen derartigen Alarmmodus und die dadurch hergestellte Aufmerksamkeit für den Fehlbetrieb vermieden werden.

Das erfindungsgemäße Konzept einer Messeinheit, die am noch nicht mit Anästhesiemittel beladenen Gas den zweiten Kennwert misst und am mit Anästhesiemittel beladenen Gas den ersten Kennwert misst und daraus über die Kalibrierungsinformation die Anästhesiemittelkonzentration bestimmt, kann grundsätzlich natürlich auch an einem einzigen Gaszweig erfolgen. Insbesondere kann dieses Konzept auch für eine Anästhesiemitteldosiervorrichtung verwendet werden, die nur einen Gaszweig mit Anästhesiemittelabgabeeinheit aufweist. Dies ist beispielsweise bei der Verwendung einer Anästhesiemitteleinspritzung möglich, bei der nicht über eine Steuerung des Gasstroms, sondern über eine Steuerung des Einspritzvolumens des Anästhesiemittels die Anästhesiemittelkonzentration geregelt wird. Für eine derartige Vorrichtungsstruktur ist das Konzept der erfindungsgemäßen Messeinheit natürlich ebenfalls anwendbar. Etwaige Anpassungen der Erfindung an eine solche Vorrichtung liegen im Bereich des fachmännischen Handelns.

Gemäß einem weiteren Aspekt der Erfindung wird zur Lösung der oben genannten Aufgabe ein Verfahren zum Bestimmen einer Anästhesiemittelkonzentration im Bereich eines Auslasses der Anästhesiemitteldosiervorrichtung vorgeschlagen. Das erfindungsgemäße Verfahren weist dabei die folgenden Schritte auf:
- Bereitstellen eines ersten und zweiten Gaszweigs, wobei der erste Gaszweig mit einer Atemgaszufuhr verbindbar ist und ausgebildet ist, ein Atemgas durch einen Anästhesiemittelverdunster zu einer Mischereinheit zu führen, und wobei der zweite Gaszweig mit der Atemgaszufuhr verbindbar ist und ausgebildet ist, das Atemgas ebenfalls zu der Mischereinheit zu führen, und wobei die Mischereinheit angeordnet und ausgebildet ist, das mit einem Anästhesiemittel angereicherte Atemgas aus dem ersten Gaszweig mit dem Atemgas aus dem zweiten Gaszweig zu mischen und das dadurch entstandene Gasgemisch einem Auslass der Anästhesiemitteldosiervorrichtung bereitzustellen;
- Messen eines ersten Kennwerts einer Gaskonzentrations-abhängigen Kenngröße zwischen Mischereinheit und Auslass mittels eines ersten Sensorelements;
- Messen eines zweiten Kennwerts der Gaskonzentrations-abhängigen Kenngröße in dem zweiten Gaszweig oder im Bereich der Atemgaszufuhr mittels eines zweiten Sensorelements;
- Bestimmen der Anästhesiemittelkonzentration zwischen Mischereinheit und Auslass basierend auf einer dem zweiten Kennwert zugeordneten Kalibrierungsinformation, insbesondere einer vorbestimmten Kalibrierungsinformation, und zumindest dem ersten Kennwert;
- Ausgeben eines Konzentrationssignals, welches die bestimmte Anästhesiemittelkonzentration indiziert;
- Umschalten zwischen der Messung des ersten Kennwerts und der Messung des zweiten Kennwerts durch das jeweils verbundene Sensorelement.

Vorteilhaft erlaubt das erfindungsgemäße Verfahren eine besonders robuste Bestimmung der Anästhesiemittelkonzentration, da nicht über einen einzigen komplexen Messvorgang die Anästhesiemittelkonzentration ermittelt wird, sondern über zwei robuste Messungen von unselektiven Kennwerten die Anästhesiemittelkonzentration erfasst werden kann. Hierdurch ist die Verwendung vergleichsweise einfacher Sensoren, wie beispielsweise Temperatursensoren, Dichtesensoren, Ultraschallsensoren und/oder optischer Sensoren, für die Umsetzung des erfindungsgemäßen Verfahrens möglich.

In einer besonders bevorzugten Ausführungsform weist das erfindungsgemäße Verfahren weiterhin die Schritte auf:
- Empfangen des ausgegebenen Konzentrationssignals;
- Regeln der Anreicherung des Atemgases mit Anästhesiemittel in dem ersten Gaszweig basierend auf einem Vergleich zwischen einer vorbestimmten Soll-Anästhesiemittelkonzentration und der bestimmten Anästhesiemittelkonzentration.

Die vorbestimmte Soll-Anästhesiemittelkonzentration wird beispielsweise über eine Benutzerschnittstelle der Anästhesiemitteldosiervorrichtung durch eine Benutzereingabe empfangen. Alternativ ist die vorbestimmte Soll-Anästhesiemittelkonzentration automatisiert vorgeben, beispielsweise automatisiert abhängig von Messwerten anderer medizinischer Geräte, wie beispielsweise eines mit der Anästhesiemitteldosiervorrichtung verbundenen Anästhesiegerätes vorgegeben.

Die Erfindung soll nun anhand von in den Figuren schematisch dargestellten, vorteilhaften Ausführungsbeispielen näher erläutert werden. Von diesen zeigen im Einzelnen:
- Fig. 1: eine schematische Darstellung eines ersten Beispiels einer nicht erfindungsgemäßen Anästhesiemitteldosiervorrichtung;
- Fig. 2: eine schematische Darstellung eines zweiten Beispiels einer nicht erfindungsgemäßen Anästhesiemitteldosiervorrichtung;
- Fig. 3: eine schematische Darstellung eines dritten Ausführungsbeispiels einer erfindungsgemäßen Anästhesiemitteldosiervorrichtung;
- Fig. 4: eine schematische Darstellung eines vierten Ausführungsbeispiels der erfindungsgemäßen Anästhesiemitteldosiervorrichtung;
- Fig. 5: ein Flussdiagramm eines ersten Ausführungsbeispiels eines Verfahrens gemäß einem weiteren Aspekt der Erfindung.

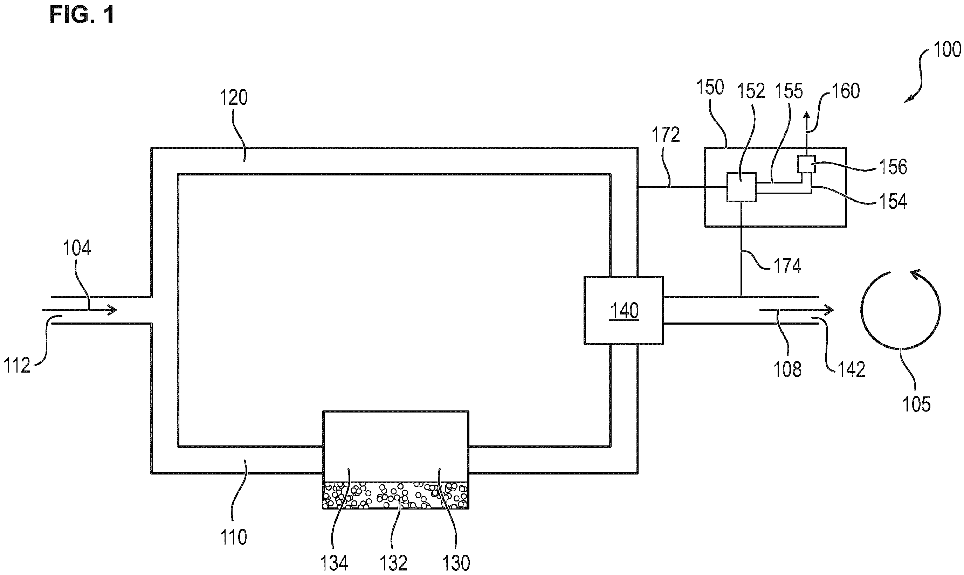

Fig. 1 zeigt eine schematische Darstellung eines nicht erfindungsgemäßen Beispiels einer nicht erfindungsgemäßen Anästhesiemitteldosiervorrichtung 100.

Die Anästhesiemitteldosiervorrichtung 100 umfasst einen ersten Gaszweig 110, einen zweiten Gaszweig 120, einen Anästhesiemittelverdunster 130, eine Mischereinheit 140 und eine Messeinheit 150.

Die Anästhesiemitteldosiervorrichtung 100 ist zum Einstellen einer Anästhesiemittelkonzentration ausgebildet in einem einem Anästhesieatemkreis 105 bereitzustellendem Gasgemisch 108.

Der erste Gaszweig 110 ist mit einer Atemgaszufuhr 112 verbindbar und ausgebildet, ein Atemgas 104 durch den Anästhesiemittelverdunster 130 zu der Mischereinheit 140 zu führen.

Der zweite Gaszweig 120 ist mit der Atemgaszufuhr 112 verbindbar und ausgebildet, das Atemgas 104 ebenfalls zu der Mischereinheit 140 zu führen. Die beiden Gaszweige 110, 120 weisen jeweils einen Hohlraum auf, durch den das Atemgas 104 jeweils geführt wird und der durch eine rohrförmige Wandung begrenzt ist.

Der Anästhesiemittelverdunster 130 ist angeordnet und ausgebildet, das Atemgas 104 in dem ersten Gaszweig 110 mit einem Anästhesiemittel 132 anzureichern. Das Anästhesiemittel 132 befindet sich dafür vorzugsweise in einer Verdunsterkammer 134. Typischerweise liegt das Anästhesiemittel 132 innerhalb der Verdunsterkammer 134 sowohl flüssig als auch gasförmig vor. Die Struktur einer derartigen Verdunsterkammer ist allgemein bekannt und wird daher im Folgenden nicht im Detail erläutert.

Die Mischereinheit 140 ist angeordnet und ausgebildet, das mit dem Anästhesiemittel 132 angereicherte Atemgas 104 aus dem ersten Gaszweig 110 mit dem Atemgas 104 aus dem zweiten Gaszweig 120 zu mischen und das dadurch entstandene Gasgemisch 108 einem Auslass 142 der Anästhesiemitteldosiervorrichtung 100 bereitzustellen. In dem dargestellten Ausführungsbeispiel ist die Mischereinheit 140 beabstandet von dem Auslass 142 angeordnet. In einem nicht dargestellten Ausführungsbeispiel umfasst die Mischereinheit den Auslass der Anästhesiemitteldosiervorrichtung.

Die Messeinheit 150 ist zum Bestimmen einer Anästhesiemittelkonzentration im Bereich des Auslasses 142 der Anästhesiemitteldosiervorrichtung 100 ausgebildet. Dabei ist sie ausgebildet, durch mindestens ein Sensorelement 152 einen ersten Kennwert 154 einer Gaskonzentrations-abhängigen Kenngröße zwischen Mischereinheit 140 und Auslass 142 zu messen und einen zweiten Kennwert 155 der Gaskonzentrations-abhängigen Kenngröße in dem zweiten Gaszweig 120 oder im Bereich der Atemgaszufuhr 112 zu messen. In dem dargestellten Beispiel wird der erste Kennwert 154 in Flussrichtung direkt vor dem Auslass 142 gemessen und der zweite Kennwert 155 wird im zweiten Gaszweig 120 im Bereich vor der Mischereinheit 140 gemessen. Bei der Gaskonzentrations-abhängigen Kenngröße handelt es sich um die Wärmeleitfähigkeit des untersuchten Gasgemisches. In einem nicht dargestellten Beispiel handelt es sich bei der Gaskonzentrations-abhängigen Kenngröße um die Dichte des untersuchten Gasgemisches oder um ein optisches Spektrum des untersuchten Gasgemisches oder dergleichen. Die Messeinheit 150 in dem dargestellten Beispiel umfasst genau ein Sensorelement 152.

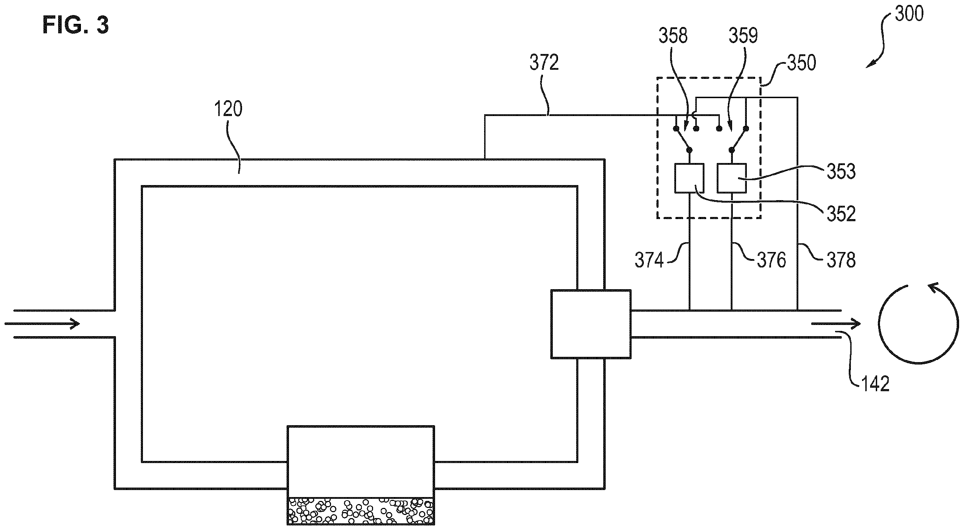

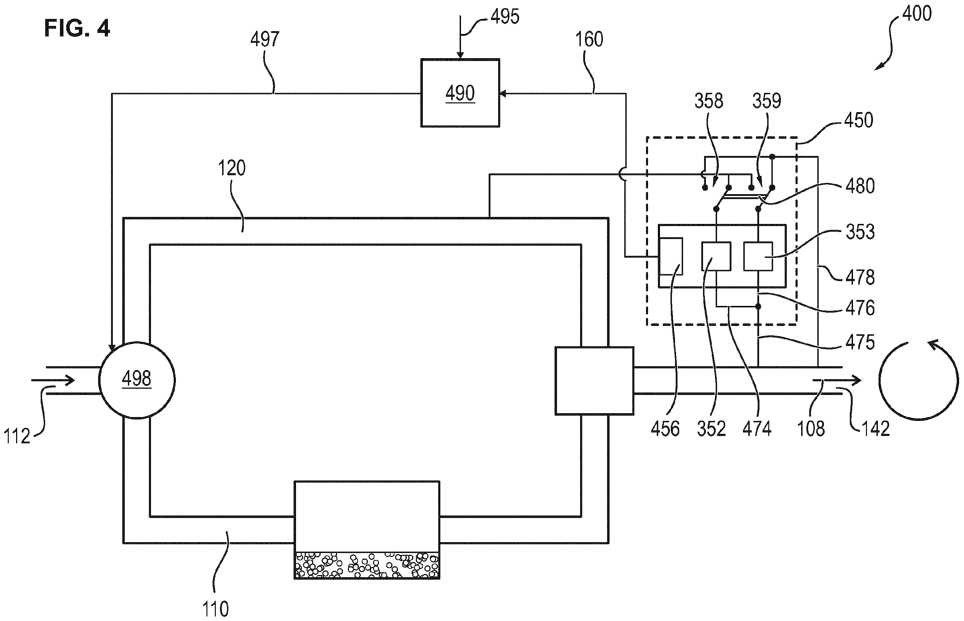

Im Rahmen der nachfolgenden, erfindungsgemäßen Figuren 3 und 4 sind Messeinheiten dargestellt, die genau zwei Sensorelemente umfassen. In nicht dargestellten Ausführungsbeispielen umfasst die erfindungsgemäße Messeinheit drei oder mehr Sensorelemente zum Messen von Kennwerten der Gaskonzentrations-abhängigen Kenngröße.

Erfindungsgemäß ist die Messeinheit 150 ausgebildet, abhängig von einer dem zweiten Kennwert 155 zugeordneten Kalibrierungsinformation, insbesondere einer vorbestimmten Kalibrierungsinformation, und zumindest dem ersten Kennwert 154 die Anästhesiemittelkonzentration zwischen Mischereinheit 140 und Auslass 142 zu bestimmen und ein entsprechendes Konzentrationssignal 160 auszugeben.

In dem dargestellten Beispiel wird die Gaskonzentrations-abhängige Kenngröße im Rahmen des ersten Kennwerts 154 für ein Gasgemisch bestimmt, das sowohl das über die Atemgaszufuhr 112 zugeführte Atemgas, als auch das über den Anästhesiemittelverdunster 130 zugeführte Anästhesiemittel umfasst. Im Rahmen des zweiten Kennwerts 155 wird die Gaskonzentrations-abhängige Kenngröße für ein Gasgemisch bestimmt, das nur das ihr über die Atemgaszufuhr 112 zugeführte Atemgas in dem zweiten Gaszweig 120 umfasst. Durch das Bestimmen der beiden Kennwerte 154, 155 kann der Einfluss des Anästhesiemittels auf die gemessene Wärmeleitfähigkeit bestimmt werden. Für die Messung der Wärmeleitfähigkeit wird vorliegend, wie auf dem Gebiet der Wärmeleitfähigkeitsmessung üblich, das Gas auf eine Messtemperatur erwärmt, die die Umgebungstemperatur übersteigt, beispielsweise mehr als 45 °C, insbesondere auf etwa 50 °C. Hierdurch wird eine Ungenauigkeit aufgrund einer Temperaturschwankung vermieden. Anhand des zweiten Kennwerts 155 wird über die Kalibrierungsinformation auf das zugeführte Atemgas geschlossen, insbesondere wird darauf geschlossen, ob ein Sauerstoff-Stickstoff-Gemisch oder ein Sauerstoff-Lachgas-Gemisch verwendet wurde. Die Kalibrierungsinformation umfasst in dem dargestellten Beispiel eine Mehrzahl von Atemgaszusammensetzungs-abhängigen Kalibrierungskurven, nämlich eine jeweilige Atemgaszusammensetzungs-abhängige Kalibrierungskurve für ein Sauerstoff-Stickstoff-Gemisch und für ein Sauerstoff-Lachgas-Gemisch. Anhand des bestimmten ersten Kennwerts wird daraufhin die Anästhesiemittelkonzentration bestimmt. In einem nicht dargestellten Ausführungsbeispiel ergibt sich über die aus dem zweiten Kennwert bekannte Zusammensetzung des zugeführten Atemgases aus dem ersten Kennwert das verwendete Anästhesiemittel. Hierfür wird vorzugsweise ebenfalls auf die Kalibrierungsinformation zurückgegriffen.

Die Kalibrierungsinformation indiziert dabei beispielsweise die Abhängigkeit zwischen der Wärmeleitfähigkeit in dem zweiten Gaszweig oder im Bereich der Atemgaszufuhr und dem Verhältnis der Zusammensetzung der bekannten, beispielsweise vorbestimmten Komponenten des zugeführten Atemgases. So kann über die Messung des zweiten Kennwerts durch die Messeinheit dieses Verhältnis ermittelt werden. Weiterhin indiziert die Kalibrierungsinformation vorzugsweise für das ermittelte Verhältnis der Zusammensetzung des zugeführten Atemgases die Abhängigkeit zwischen Wärmeleitfähigkeit in dem Bereich zwischen Mischereinheit und Auslass und der Anästhesiemittelkonzentration für das bekannte, beispielsweise vorbestimmte, Anästhesiemittel. So kann durch den ersten Kennwert anhand der dem zweiten Kennwert zugeordneten Kalibrierungsinformation die Anästhesiemittelkonzentration bestimmt werden.

Die verwendete Kalibrierungsinformation wird zum Beispiel ermittelt, indem ein linearer und ein nicht-linearer Zuwachs in der Wärmeleitung in Abhängigkeit von der Zumischung eines weiteren Gases als eine Kalibrierungskurve hinterlegt werden.

Vorzugsweise besteht die Kalibrierungsinformation aus einer ersten Kennlinienkurve, durch die abhängig vom zweiten Kennwert die Zusammensetzung des zugeführten Gases bestimmt wird. Dieser Information kann eine zweite Kalibrierungskurve zugeordnet werden, die eine Abhängigkeit zwischen erstem Kennwert oder Differenz aus erstem und zweitem Kennwert und Anästhesiemittelkonzentration beschreibt. Die zweite Kalibrierungskurve wird dabei vorzugsweise abhängig von dem zweiten Kennwert aus einer Gruppe von zweiten Kalibrierungskurven ausgewählt.

Die Kalibrierungsinformation kann in Form von einer oder mehrerer Datentabellen und/oder in Form von einer oder mehrerer Diagramme oder Kennlinien oder Funktionen vorliegen. Unter Kennlinien sind erfindungsgemäß auch Kurven zu verstehen. In dem dargestellten Beispiel umfasst die Messeinheit 150 ein Speichermodul (nicht dargestellt), in dem die Kalibrierungsinformation in Form von Kennlinien hinterlegt ist. Weiterhin umfasst die Messeinheit 150 in dem dargestellten Ausführungsbeispiel einen Prozessor 156, der abhängig von der Kalibrierungsinformation und den beiden Kennwerten in erfindungsgemäßer Weise die Anästhesiemittelkonzentration bestimmt und das entsprechende Konzentrationssignal 160 ausgibt.

In einem nicht dargestellten Ausführungsbeispiel wird die Anästhesiemittelkonzentration basierend auf einer Dichtemessung der Dichte des am jeweiligen Sensor vorbeigeführten Gases bestimmt. Die Kalibrierungsinformation und das Verfahren zum Bestimmen der Anästhesiemittelkonzentration aus der Dichtemessung können beispielsweise analog zu dem in den vorhergehenden Absätzen zur Messung der Wärmeleitfähigkeit beschriebenen Vorgehen vorgesehen und durchgeführt werden. Vorzugsweise wird erfindungsgemäß auch der zweite Kennwert durch eine Dichtemessung über eine entsprechende Kennlinie bestimmt und basierend auf dem zweiten Kennwert wird die zugeordnete Kalibrierungsinformation verwendet um daraus zumindest basierend auf dem ersten Kennwert die Anästhesiemittelkonzentration zu bestimmen.

Das ausgegebene Konzentrationssignal zeigt einem Nutzer der Anästhesiemitteldosiervorrichtung 100 an, ob die gewünschte Anästhesiemittelkonzentration dem Anästhesieatemkreis 105 zur Verfügung gestellt wird. Dadurch indiziert das Konzentrationssignal 160 eine beispielsweise manuell auszuführende Veränderung der Anästhesiemittelkonzentration über den Anästhesiemittelverdunster 130, falls eine gewünschte Anästhesiemittelkonzentration noch nicht durch die Anästhesiemitteldosiervorrichtung 100 erreicht wurde.

In einem nicht dargestellten Ausführungsbeispiel ist der Prozessor der Messeinheit räumlich getrennt von dem mindestens einen Sensorelement angeordnet. Weiterhin kann die erfindungsgemäße Messeinheit aus mehreren räumlich getrennten Modulen bestehen oder beispielsweise von einem einzigen Gehäuse oder einer einzigen Einfassung einer Anästhesievorrichtung umgeben sein.

In dem dargestellten Beispiel wird die Verbindung zwischen dem jeweiligen Gaszweig und der Messeinheit 150 über einen jeweiligen Messgasführungskanal 172, 174 erreicht. In dem jeweiligen Messgasführungskanal 172, 174 erfolgt die Messung der beiden Kennwerte 154, 155. Über den ersten Messgasführungskanal 172 erfolgt dabei die Messung des zweiten Kennwerts 155 und über den zweiten Messgasführungskanal 174 erfolgt die Messung des ersten Kennwerts 154. Dabei sind die Messgasführungskanälen 172, 174 derart pneumatische in die Atemgasführung der Anästhesiemitteldosiervorrichtung 100 eingebunden, dass temporäre Druckschwankungen innerhalb des ersten und/oder zweiten Gaszweigs zumindest teilweise auch in entsprechender Weise innerhalb der beiden Messgasführungskanäle 172, 174 vorliegen. Solche temporären Druckschwankungen werden also auch innerhalb der beiden Messgasführungskanälen 172, 174 vorliegen, jedoch aufgrund der pneumatischen Struktur in möglicherweise reduzierter Weise. Der jeweilige Messgasführungskanal 172, 174 hat einen kleineren Querschnitt als der jeweilige Gaszweig, vorzugsweise höchstens ein Viertel des Gaszweig-Querschnitts, insbesondere höchstens ein Zehntel des Gaszweig-Querschnitts, besonders bevorzugt höchstens ein Zwanzigstel des Gaszweig-Querschnitts. Hierbei wird der Bereich zwischen Mischereinheit 140 und Auslass 142 auch als ein Gaszweig der Anästhesiemitteldosiervorrichtung 100 bezeichnet.

In einem nicht dargestellten Ausführungsbeispiel wird der zweite Kennwert im Bereich der Atemgaszufuhr gemessen. Im Bereich der Atemgaszufuhr ist das Atemgas ähnlich wie im Bereich des zweiten Gaszweigs nicht mit einem Anästhesiemittel angereichert, so dass in diesem nicht dargestellten Ausführungsbeispiel analog zu dem beschriebenen Vorgehen bei der Bestimmung der Anästhesiemittelkonzentration vorgegangen werden kann.

Besonders vorteilhaft an der Messung des zweiten Kennwerts im Bereich des zweiten Gaszweigs 120 ist die dadurch ermöglichte geringe Länge der beiden Messgasführungskanäle 172, 174 und das damit verbundene geringe Puffervolumen von aus dem entsprechenden Gaszweig abgeführtem Messgas innerhalb der Messgasführungskanälen 172, 174. Hierdurch können beispielsweise Druckschwankungen oder Schwankungen in der Anästhesiemittelkonzentration besonders schnell durch die Messeinheit 150 erfasst werden.

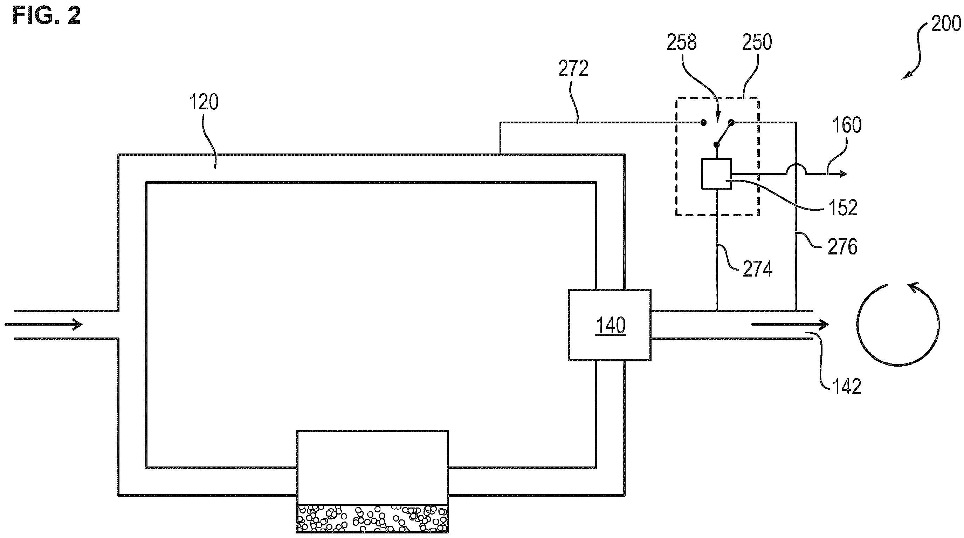

Eine mögliche Struktur der Messeinheit, die veranschaulicht wie die beiden Kennwerte 154, 155 durch ein einziges Sensorelement 152 gemessen werden können, wird im Rahmen von Fig. 2 gezeigt.

Die verschiedenen Teile der Anästhesiemitteldosiervorrichtung 100 sind in dem dargestellten Beispiel in einem gemeinsamen Gehäuse (nicht dargestellt) angeordnet, welches an den Anästhesieatemkreis 105 angeschlossen werden kann. In einem anderen Ausführungsbeispiel ist die erfindungsgemäße Anästhesiemitteldosiervorrichtung in ein Anästhesiegerät mit einem entsprechenden Anästhesieatemkreis integriert. In einem weiteren Ausführungsbeispiel sind die verschiedenen Teile der erfindungsgemäßen Anästhesiemitteldosiervorrichtung zumindest teilweise in verschiedenen Gehäusen angeordnet.

Weitere Bestandteile einer typischen Anästhesiemitteldosiervorrichtung, wie beispielsweise ein Ventil, eine Dichtung, ein Gasflussmessgerät oder dergleichen, können ebenfalls Bestandteile der erfindungsgemäßen Anästhesiemitteldosiervorrichtung sein, wie sich dies unmittelbar aus der vorliegenden Beschreibung der erfindungsgemäßen Lehre für einen Fachmann auf dem Gebiet ergibt.

Fig. 2 zeigt eine schematische Darstellung eines zweiten Beispiels einer nicht erfindungsgemäßen Anästhesiemitteldosiervorrichtung 200.

Die dargestellte Anästhesiemitteldosiervorrichtung 200 unterscheidet sich hauptsächlich dadurch von der in Fig. 1 dargestellten Anästhesiemitteldosiervorrichtung 100, dass die Messeinheit 250 einen Schalter 258 aufweist, mit dem zwischen der Messung des ersten Kennwerts und der Messung des zweiten Kennwerts durch das eine Sensorelement 152 umgeschaltet werden kann.

Das Umschalten des Schalters 258 erfolgt vorzugsweise in regelmäßigen zeitlichen Abständen, wobei die regelmäßigen zeitlichen Abstände vorzugsweise weniger als 60 Sekunden, insbesondere weniger als 40 Sekunden, besonders bevorzugt weniger als 20 Sekunden betragen. Hierdurch kann die Messung der beiden Kennwerte mit einem lediglich geringen zeitlichen Versatz erfolgen. Ein geringer zeitlicher Versatz zwischen den beiden Messungen ermöglicht eine besonders genaue Bestimmung der Anästhesiemittelkonzentration durch die Messeinheit 250, da Druck- und/oder Konzentrationsschwankungen bei beiden Messungen gleichmäßig berücksichtigt werden können.

Die dargestellten Messgasführungskanäle 272, 274, 276 führen dazu, dass das Sensorelement 152 für die beiden Messungen in unterschiedliche Richtungen von dem jeweils zu messenden Gasgemisch durchströmt oder angeströmt wird. So wird bei der Messung des zweiten Kennwerts in dem zweiten Gaszweig 120 der zu messende Gasstrom durch den ersten Messgasführungskanal 272 zu dem Sensorelement 152 und daraufhin über den zweiten Messgasführungskanal 274 zu dem Auslass 142 geführt. Hingegen bei der Messung des ersten Kennwerts wird der zu messende Gasstrom zwischen Mischereinheit 140 und Auslass 142 durch den zweiten Messgasführungskanal 274 entnommen, durch das Sensorelement 152 geführt und daraufhin über den Schalter 258 und den dritten Messgasführungskanal 276 zurück zu dem Auslass 142 geführt.

Die Verarbeitung der beiden Kennwerte ist in dem dargestellten Ausführungsbeispiel nicht dargestellt. Diese Verarbeitung führt zu dem ausgegebenen Konzentrationssignal 160.

Der Schalter 258 ist in dem dargestellten Ausführungsbeispiel ein elektrisch betriebener Kippschalter. Dieser Kippschalter wird über die Messeinheit 250 gesteuert. Hierdurch kann die Messeinheit 250 direkt über die Schalterstellung des Schalters 258 auf den aktuell gemessenen Kennwert oder den aktuell zu messenden Kennwert schließen und das Messergebnis entsprechend verarbeiten. In einem nicht dargestellten Ausführungsbeispiel handelt es sich bei dem Schalter um einen manuell zu betätigenden Schalter. Neben dem Kippschalter sind dem Fachmann auf diesem Gebiet eine Vielzahl weiterer Schalter für derartige pneumatische Anwendungen bekannt und werden daher im Folgenden nicht im Detail erläutert. In einem weiteren nicht dargestellten Ausführungsbeispiel wird zwischen der Messung des ersten Kennwerts und der Messung des zweiten Kennwerts durch eine Veränderung der räumlichen Position des mindestens einen Sensorelementes umgeschaltet. Hierdurch ist ein Umschalten zwischen den beiden Messungen möglich, ohne dass an der Führung des zu messenden Gases etwas verändert werden muss.

Vorzugsweise ist der Schalter 258 derart ausgebildet, dass während eines Umschaltvorgangs derjenige Messgasführungskanal, der nicht mehr zum Messen eines Kennwerts verwendet wird, derart abgedichtet ist, dass kein Gas im Bereich des Schalters 258 austreten kann. Hierdurch wird sichergestellt, dass in dem entsprechenden Gaszweig der Anästhesiemitteldosiervorrichtung 200 kein weiteres Gas zum Messen des aktuell nicht zu messenden Kennwerts entnommen wird.

Fig. 3 zeigt eine schematische Darstellung eines dritten Ausführungsbeispiels einer erfindungsgemäßen Anästhesiemitteldosiervorrichtung 300.

Die Anästhesiemitteldosiervorrichtung 300 unterscheidet sich dadurch von der in Fig. 2 dargestellten Anästhesiemitteldosiervorrichtung 200, dass die Messeinheit 350 zwei Sensorelemente 352, 353 aufweist.

Jedem der beiden Sensorelemente 352, 353 ist ein jeweiliger Schalter 358, 359 zugeordnet, über den eingestellt wird, welcher Kennwert der beiden zu messenden Kennwerte durch das dem jeweiligen Schalter 358, 359 zugeordnete Sensorelement 352, 353 gemessen wird. Durch die dargestellte Verschaltung können beide Sensorelemente 352, 353 abhängig von der vorliegenden Schalterstellung des jeweiligen Schalters 358, 359 beide Kennwerte messen.

Das erste Sensorelement 352 ist dem ersten Schalter 358 zugeordnet und das zweite Sensorelement 353 ist dem zweiten Schalter 359 zugeordnet. Die jeweiligen Messgasführungskanäle sind derart angeordnet, dass die beiden Sensorelemente 352, 353 zeitgleich denselben Kennwert bestimmen können. Eine derartige redundante Messung kann beispielsweise zur Überprüfung der Funktionsfähigkeit beider Sensorelemente vorteilhaft sein.

In der dargestellten Schalterstellung der beiden Schalter 358, 359 ist eine gleichzeitige Messung des ersten und zweiten Kennwerts möglich. So wird über den ersten Messgasführungskanal 372 das für die Messung benötigte Gas aus dem zweiten Gaszweig 120 zu dem ersten Schalter 358 und dadurch zu dem ersten Sensorelement 352 geführt. Von dem ersten Sensorelement wird es über den zweiten Messgasführungskanal 374 in den Bereich des Auslasses 142 der Anästhesiemitteldosiervorrichtung 300 geführt. Für die Messung des ersten Kennwertes wird das zu messende Gas aus dem Bereich des Auslasses 142 über den dritten Messgasführungskanal 376 zu dem zweiten Sensorelement 353 geführt und über den zweiten Schalter 359 und einen vierten Messgasführungskanal 378 zu dem Auslass 142 geleitet.

Die beiden Schalter 358, 359 werden von der Messeinheit 350 gesteuert und können dabei unabhängig voneinander ihre jeweilige Schalterstellung verändern.

Die Ausgabe des entsprechenden Konzentrationssignals ist aus Gründen der Übersichtlichkeit nicht in Fig. 3 dargestellt.

Fig. 4 zeigt eine schematische Darstellung eines vierten Ausführungsbeispiels der erfindungsgemäßen Anästhesiemitteldosiervorrichtung 400.

Die Anästhesiemitteldosiervorrichtung 400 unterscheidet sich von der in Fig. 3 dargestellten Anästhesiemitteldosiervorrichtung 300 dadurch, dass die beiden Schalter 358, 359 miteinander über ein Koppelelement 480, insbesondere ein mechanisches Koppelelement 480, gekoppelt sind. Über dieses Koppelelement 480 wird sichergestellt, dass ein Wechsel einer ersten Schalterstellung des ersten Schalters 358 auch einen Wechsel der zweiten Schalterstellung des zweiten Schalters 359 bewirkt. Hierdurch messen die beiden Sensorelemente 352, 353 nicht gleichzeitig denselben Kennwert, sondern wechseln stets zwischen einem Zustand, in dem das erste Sensorelement 352 den ersten Kennwert und das zweite Sensorelement 353 den zweiten Kennwert misst, und einem Zustand, in dem das erste Sensorelement 352 den zweiten Kennwert und das zweite Sensorelement 353 den ersten Kennwert misst.

Die gleichzeitige Messung der beiden Kennwerte ermöglicht eine besonders präzise Bestimmung der Anästhesiemittelkonzentration im Bereich des Auslasses 142 der Anästhesiemitteldosiervorrichtung 400. Weiterhin führt der Wechsel des jeweils einen Kennwert messenden Sensorelements zu einer redundanten Messung der beiden Kennwerte und dadurch zu einem zuverlässig überprüfbaren Gerätebetrieb, da ein Fehler eines Sensorelementes besonders schnell erkannt werden kann.

Weiterhin unterscheidet sich die Anästhesiemitteldosiervorrichtung 400 dadurch von den vorher beschriebenen Ausführungsbeispielen, dass sie weiterhin eine Steuereinheit 490 aufweist, die das von dem Prozessor 456 der Messeinheit 450 bestimmte und ausgegebene Konzentrationssignal 160 empfängt. Weiterhin ist die Steuereinheit 490 dazu ausgebildet, ein Eingabesignal 495 zu empfangen, das eine vorbestimmte Soll-Anästhesiemittelkonzentration indiziert. Das Eingabesignal 495 kann beispielsweise durch eine Benutzereingabe an einer Benutzerschnittstelle oder durch eine zentrale Verarbeitungseinheit eines mit der Anästhesiemitteldosiervorrichtung 400 verbundenen Anästhesiegerätes bereitgestellt werden. Weiter ist die Steuereinheit 490 dazu ausgebildet, basierend auf einem Vergleich zwischen der vorbestimmten Soll-Anästhesiemittelkonzentration und der durch das Konzentrationssignal 160 indizierten bestimmten Anästhesiemittelkonzentration ein Steuersignal 497 an eine Flusssteuerungseinheit 498 zu senden. Die Flusssteuerungseinheit 498 ist dazu ausgebildet, den Gasfluss aus der Atemgaszufuhr 112 in den ersten und zweiten Gaszweig 110, 120 zu verteilen. Über das Steuersignal 497 ist die Steuereinheit 490 dazu ausgebildet, die Flusssteuerungseinheit 498 derart anzusteuern, dass basierend auf dem Vergleich der vorbestimmten Soll-Anästhesiemittelkonzentration und der bestimmten Anästhesiemittelkonzentration die Anreicherung des Atemgases mit Anästhesiemittel in dem ersten Gaszweig 110 gesteuert wird. Beispielsweise kann bei einer bestimmten Anästhesiemittelkonzentrationen, die unterhalb der Soll-Anästhesiemittelkonzentration liegt, ein Gasfluss durch den ersten Gaszweig 110 vergrößert und dadurch der Anteil des mit Anästhesiemittel angereicherten Atemgases im Gasgemisch 108 am Auslass 142 erhöht werden.

In einem nicht dargestellten Ausführungsbeispiel ist die Steuereinheit weiterhin dazu ausgebildet, basierend auf der gesteuerten Aufteilung der Gasflüsse durch den ersten und zweiten Gaszweig ein Laufzeitkorrektursignal an die Messeinheit auszugeben, das einen Unterschied in der aktuellen Laufzeit für Gas in dem ersten und zweiten Gaszweig anzeigt. Weiterhin ist die Messeinheit dazu ausgebildet, basierend auf dem Laufzeitkorrektursignal die Anästhesiemittelkonzentration zu bestimmen. So kann vorteilhaft ein Unterschied in der Zusammensetzung des zugeführten Atemgases während der gleichzeitigen Messung der beiden Kennwerte berücksichtigt werden. Beispielsweise können die Kennwerte von der Messeinheit über einen vorbestimmten Zeitraum gespeichert und im Falle einer Zusammensetzungsveränderung nach dem vorbestimmten Zeitraum korrigiert werden, wobei erst nach dem vorbestimmten Zeitraum, vorzugsweise mindestens 2 Sekunden, insbesondere mindestens 5 Sekunden, besonders bevorzugt etwa 10 Sekunden, die Anästhesiemittekonzentration bestimmt und an die Steuereinheit ausgegeben wird. In einem alternativen nicht dargestellten Ausführungsbeispiel ist die Steuereinheit dazu ausgebildet, die beschriebene Laufzeitkorrektur mit dem empfangenen Konzentrationssignal der Messeinheit zu verrechnen, wobei das Konzentrationssignal vorzugsweise eine Anzahl von gemessenen ersten und zweiten Kennwerten indiziert, die ein Verrechnen der Kennwerte mit der Laufzeitkorrektur zum Bestimmen der Anästhesiemittelkonzentration ermöglichen.

Schließlich unterscheidet sich die Anästhesiemitteldosiervorrichtung 400 dadurch von der Anästhesiemitteldosiervorrichtung 300, dass der zweite und dritte Messgasführungskanal 474, 476 einen gemeinsamen Anschluss 475 an die Gasführung im Bereich des Auslasses 142 aufweisen. Dies wird dadurch ermöglicht, dass der erste Kennwert dadurch ermittelt wird, dass das entsprechende Messgas über den vierten Messgasführungskanal 478 zu dem zweiten Sensorelement 353 geführt wird, so dass, anders als bei der Anästhesiemitteldosiervorrichtung 300, der zweite und dritte Messgasführungskanal 474, 476 in die gleiche Richtung von einem Messgas durchströmt werden.

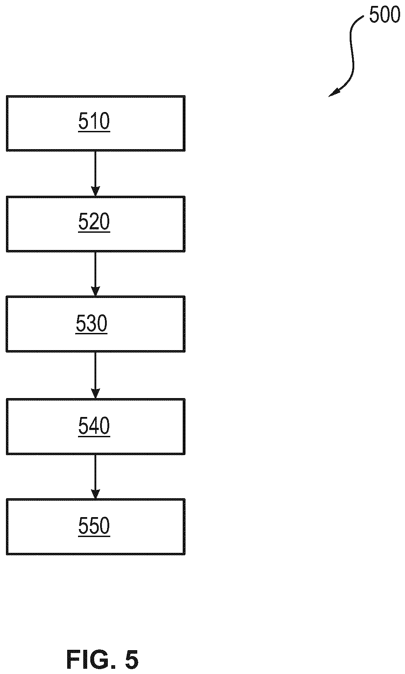

Fig. 5 zeigt ein Flussdiagramm eines ersten Ausführungsbeispiels eines Verfahrens 500 gemäß einem weiteren Aspekt der Erfindung.

Das Verfahren 500 ist zum Bestimmen einer Anästhesiemittelkonzentration im Bereich eines Auslasses einer Anästhesiemitteldosiervorrichtung ausgebildet. Dabei weist es die im Folgenden beschriebenen Schritte 510 bis 550 auf.

Ein erster Schritt 510 umfasst ein Bereitstellen eines ersten und zweiten Gaszweigs, wobei der erste Gaszweig mit einer Atemgaszufuhr verbindbar ist und ausgebildet ist, ein Atemgas durch einen Anästhesiemittelverdunster zu einer Mischereinheit zu führen, und wobei der zweite Gaszweig mit der Atemgaszufuhr verbindbar ist und ausgebildet ist, das Atemgas ebenfalls zu der Mischereinheit zu führen. Dabei ist die Mischereinheit angeordnet und ausgebildet, das mit einem Anästhesiemittel angereicherte Atemgas aus dem ersten Gaszweig mit dem Atemgas aus dem zweiten Gaszweig zu mischen und das dadurch entstandene Gasgemisch einem Auslass der Anästhesiemitteldosiervorrichtung bereitzustellen.

Ein nächster Schritt 520 umfasst ein Messen eines ersten Kennwerts einer Gaskonzentrations-abhängigen Kenngröße zwischen Mischereinheit und Auslass.

Ein darauffolgender Schritt 530 umfasst ein Messen eines zweiten Kennwerts der Gaskonzentrations-abhängigen Kenngröße in dem zweiten Gaszweig oder im Bereich der Atemgaszufuhr.

Ein weiterer Schritt 540 umfasst ein Bestimmen der Anästhesiemittelkonzentration zwischen Mischereinheit und Auslass basierend auf einer dem zweiten Kennwert zugeordneten Kalibrierungsinformation und zumindest dem ersten Kennwert.

Ein abschließender Schritt 550 umfasst ein Ausgeben eines Konzentrationssignals, welches die bestimmte Anästhesiemittelkonzentration indiziert.

Der Schritt 510 wird vorzugsweise bei der Herstellung der erfindungsgemäßen Anästhesiemitteldosiervorrichtung ausgeführt und daraufhin nicht wiederholt. Die Schritte 520 und 530 können in unterschiedlicher Reihenfolge ausgeführt werden und sind grundsätzlich unabhängig voneinander. Dabei sollte jedoch für ein möglichst genaues Bestimmten der Anästhesiemittelkonzentration ein geringer zeitlicher Abstand zwischen der Ausführung der Schritte 520 und 530 liegen. Die Schritte 540 und 550 werden in dieser Reihenfolge ausgeführt, nachdem die beiden Kennwerte im Rahmen der Schritte 520 und 530 gemessen wurden.

Das Messen im Rahmen der Schritte 520 und 530 erfolgt vorzugsweise in regelmäßigen zeitlichen Abständen. Die regelmäßigen zeitlichen Abstände umfassen vorzugsweise zeitliche Intervalle, die weniger als 60 Sekunden betragen, insbesondere weniger als 40 Sekunden, besonders bevorzugt weniger als 20 Sekunden. Alternativ oder ergänzend kann nach dem Ausgeben des Konzentrationssignals im Rahmen von Schritt 550 wieder das Messen der beiden Kennwerte und mithin die Schritte 520 und 530 ausgeführt werden.

In einer besonders bevorzugten Variante dieser Ausführungsform umfasst das Verfahren weiterhin die folgenden Schritte, die vorzugsweise nach dem Schritt 550 ausgeführt werden:
- Empfangen des ausgegebenen Konzentrationssignals;
- Regeln der Anreicherung des Atemgases mit Anästhesiemittel in dem ersten Gaszweig basierend auf einem Vergleich zwischen einer vorbestimmten Soll-Anästhesiemittelkonzentration und der bestimmten Anästhesiemittelkonzentration.

In dieser Variante kann die bestimmte Anästhesiemittelkonzentration direkt verwendet werden, um sicherzustellen, dass die Anreicherung des Atemgases mit dem Anästhesiemittel derart erfolgt, dass die vorbestimmte Soll-Anästhesiemittelkonzentration erreicht wird.

Das Vorgeben der vorbestimmten Soll-Anästhesiemittelkonzentration kann beispielsweise über eine Benutzereingabe an einer entsprechenden Benutzerschnittstelle der Anästhesiemitteldosiervorrichtung erfolgen.

### Bezugszeichenliste

- 100, 200, 300, 400: Anästhesiemitteldosiervorrichtung
- 104: Atemgas
- 105: Anästhesieatemkreis
- 108: Gasgem isch
- 110: erster Gaszweig
- 112: Atemgaszufuhr
- 120: zweiter Gaszweig
- 130: Anästhesiemittelverdunster
- 132: Anästhesiem ittel
- 134: Verdunsterkammer
- 140: Mischereinheit
- 142: Auslass
- 150, 250, 350, 450: Messeinheit
- 152: Sensorelelement
- 154: erster Kennwert
- 155: zweiter Kennwert
- 156, 456: Prozessor
- 160: Konzentrationssignal
- 172, 272, 372: erster Messgasführungskanal
- 174, 274, 374, 474: zweiter Messgasführungskanal
- 258, 358: erster Schalter
- 276, 376, 476: dritter Messgasführungskanal
- 352: erstes Sensorelement
- 353: zweites Sensorelement
- 359: zweiter Schalter
- 378, 478: vierter Messgasführungskanal
- 475: gemeinsamer Anschluss
- 480: Kopplungselement
- 490: Steuereinheit
- 495: Eingabesignal
- 497: Steuersignal
- 498: Flusssteuerungseinheit
- 500: Verfahren
- 510, 520, 530, 540, 550: Schritte des Verfahrens

## Patentansprüche

1. Anästhesiemitteldosiervorrichtung (300, 400) mit einer Messeinheit (350, 450) zum Bestimmen einer Anästhesiemittelkonzentration im Bereich eines Auslasses (142) der Anästhesiemitteldosiervorrichtung (300, 400), aufweisend
- einen ersten Gaszweig (110), der mit einer Atemgaszufuhr (112) verbindbar ist und der ausgebildet ist, ein Atemgas (104) durch einen Anästhesiemittelverdunster (130) zu einer Mischereinheit (140) zu führen,
- einen zweiten Gaszweig (120), der mit der Atemgaszufuhr (112) verbindbar ist und der ausgebildet ist, das Atemgas (104) ebenfalls zu der Mischereinheit (140) zu führen,
- den Anästhesiemittelverdunster (130), der angeordnet und ausgebildet ist, das Atemgas (104) in dem ersten Gaszweig (110) mit einem Anästhesiemittel (132) anzureichern,
- die Mischereinheit (140), die angeordnet und ausgebildet ist, das mit dem Anästhesiemittel (132) angereicherte Atemgas (104) aus dem ersten Gaszweig (110) mit dem Atemgas (104) aus dem zweiten Gaszweig (120) zu mischen und das dadurch entstandene Gasgemisch (108) dem Auslass (142) der Anästhesiemitteldosiervorrichtung (300, 400) bereitzustellen,
- die Messeinheit (350, 450),
wobei die Messeinheit (350, 450) mindestens zwei Sensorelemente (352, 353) aufweist,
wobei ein erstes Sensorelement (352) der mindestens zwei Sensorelemente (352, 353) angeordnet und ausgebildet ist, zumindest einen ersten Kennwert (154) einer Gaskonzentrations-abhängigen Kenngröße zwischen Mischereinheit (140) und Auslass (142) zu messen und
wobei ein zweites Sensorelement (353) der mindestens zwei Sensorelemente (352, 353) angeordnet und ausgebildet ist, zumindest einen zweiten Kennwert (155) der Gaskonzentrations-abhängigen Kenngröße in dem zweiten Gaszweig (120) oder im Bereich der Atemgaszufuhr (112) zu messen,
wobei die Messeinheit (350, 450) weiter ausgebildet ist, abhängig von einer dem zweiten Kennwert (155) zugeordneten Kalibrierungsinformation und zumindest dem ersten Kennwert (154) die Anästhesiemittelkonzentration zwischen Mischereinheit (140) und Auslass (142) zu bestimmen und ein entsprechendes Konzentrationssignal (160) auszugeben,
wobei die Messeinheit (350, 450) mindestens zwei Schalter (358, 359) umfasst,
wobei das erste Sensorelement (352) der mindestens zwei Sensorelemente (352, 353) mit einem ersten Schalter (358) der mindestens zwei Schalter (358, 359) verbunden ist und
wobei das zweite Sensorelement (353) der mindestens zwei Sensorelemente (352, 353) mit einem zweiten Schalter (359) der mindestens zwei Schalter (358, 359) verbunden ist, und
wobei der erste und der zweite Schalter (358, 359) ausgebildet sind, zwischen der Messung des ersten Kennwerts (154) und der Messung des zweiten Kennwerts (155) durch das jeweils verbundene Sensorelement (352, 353) umschalten zu können.

2. Anästhesiemitteldosiervorrichtung (300, 400) gemäß Anspruch 1,
wobei die Messeinheit (350, 450) ausgebildet, abhängig von der dem zweiten Kennwert (155) zugeordneten Kalibrierungsinformation und einer Differenz zwischen erstem und zweitem Kennwert (154, 155) die Anästhesiemittelkonzentration zu bestimmen

3. Anästhesiemitteldosiervorrichtung (300, 400) gemäß mindestens einem der vorhergehenden Ansprüche,
wobei die mindestens zwei Sensorelemente (352, 353) die Kennwerte (154, 155) der Gaskonzentrations-abhängigen Kenngröße basierend auf einer Wärmeleitfähigkeitsmessung oder einer Dichtemessung messen.

4. Anästhesiemitteldosiervorrichtung (400) gemäß Anspruch 3,
wobei der erste Schalter (358) und der zweite Schalter (359) miteinander derart gekoppelt sind, dass ein Wechsel einer ersten Schalterstellung des ersten Schalters (358) auch einen Wechsel einer zweiten Schalterstellung des zweiten Schalters (359) bewirkt.

5. Anästhesiemitteldosiervorrichtung (300, 400) gemäß mindestens einem der vorhergehenden Ansprüche,
wobei die Messung des ersten Kennwerts (154) und die Messung des zweiten Kennwerts (155) durch die Messeinheit (350) im Wesentlichen zeitgleich erfolgen.

6. Anästhesiemitteldosiervorrichtung (300, 400) gemäß mindestens einem der vorhergehenden Ansprüche,
wobei die Kalibrierungsinformation eine Mehrzahl von Atemgaszusammensetzungs-abhängigen Kalibrierungskurven, insbesondere mindestens eine jeweilige Atemgaszusammensetzungs-abhängige Kalibrierungskurve für ein Sauerstoff-Stickstoff-Gemisch und für ein Sauerstoff-Lachgas-Gemisch, umfasst.

7. Anästhesiemitteldosiervorrichtung (300, 400) gemäß mindestens einem der vorhergehenden Ansprüche,
wobei die Messeinheit (350, 450) eine Anzahl von Messgasführungskanälen (172, 174) aufweist, in der die Messung des ersten und zweiten Kennwerts (154, 155) erfolgt und die in die Atemgasführung der Anästhesiemitteldosiervorrichtung (300, 400) derart pneumatisch eingebunden ist, dass temporäre Druckschwankungen innerhalb des ersten und/oder zweiten Gaszweigs (110, 120) auch in entsprechender Weise innerhalb der Anzahl von Messgasführungskanälen (172, 174) zumindest teilweise vorliegen.

8. Anästhesiemitteldosiervorrichtung (400) gemäß mindestens einem der vorhergehenden Ansprüche,
wobei die Anästhesiemitteldosiervorrichtung (400) weiterhin eine Steuereinheit (490) aufweist, die ausgebildet ist, das ausgegebene Konzentrationssignal (160) zu empfangen und basierend auf einem Vergleich zwischen einer vorbestimmten Soll-Anästhesiemittelkonzentration und der bestimmten Anästhesiemittelkonzentration die Anreicherung des Atemgases (104) mit Anästhesiemittel (132) in dem ersten Gaszweig (110) zu steuern.

9. Verfahren (500) zum Bestimmen einer Anästhesiemittelkonzentration im Bereich eines Auslasses (142) einer Anästhesiemitteldosiervorrichtung (300, 400) nach einem der vorherigen Ansprüche, aufweisend die Schritte
- Bereitstellen eines ersten und zweiten Gaszweigs (110, 120),
wobei der erste Gaszweig (110) mit einer Atemgaszufuhr (112) verbindbar ist und ausgebildet ist, ein Atemgas (104) durch einen Anästhesiemittelverdunster (130) zu einer Mischereinheit (140) zu führen, und
wobei der zweite Gaszweig (120) mit der Atemgaszufuhr (112) verbindbar ist und ausgebildet ist, das Atemgas (104) ebenfalls zu der Mischereinheit (140) zu führen, und
wobei die Mischereinheit (140) angeordnet und ausgebildet ist, das mit einem Anästhesiemittel (132) angereicherte Atemgas (104) aus dem ersten Gaszweig (110) mit dem Atemgas (104) aus dem zweiten Gaszweig (120) zu mischen und das dadurch entstandene Gasgemisch (108) einem Auslass (142) der Anästhesiemitteldosiervorrichtung (300, 400) bereitzustellen;
- Messen eines ersten Kennwerts (154) einer Gaskonzentrations-abhängigen Kenngröße zwischen Mischereinheit (140) und Auslass (142) mittels eines ersten Sensorelements (352);
- Messen eines zweiten Kennwerts (155) der Gaskonzentrations-abhängigen Kenngröße in dem zweiten Gaszweig (120) oder im Bereich der Atemgaszufuhr (112) mittels eines zweiten Sensorelements (353);
- Bestimmen der Anästhesiemittelkonzentration zwischen Mischereinheit (140) und Auslass (142) basierend auf einer dem zweiten Kennwert (155) zugeordneten Kalibrierungsinformation und zumindest dem ersten Kennwert (154); und
- Ausgeben eines Konzentrationssignals (160), welches die bestimmte Anästhesiemittelkonzentration indiziert, und
- Umschalten zwischen der Messung des ersten Kennwerts (154) und der Messung des zweiten Kennwerts (155) durch das jeweils verbundene Sensorelement (352, 353).

10. Verfahren (500) gemäß Anspruch 9, weiterhin aufweisend die Schritte
- Empfangen des ausgegebenen Konzentrationssignals (160);
- Regeln der Anreicherung des Atemgases (104) mit Anästhesiemittel (132) in dem ersten Gaszweig (110) basierend auf einem Vergleich zwischen einer vorbestimmten Soll-Anästhesiemittelkonzentration und der bestimmten Anästhesiemittelkonzentration.

## Claims

1. An anesthetic dosing device (300, 400) having a measuring unit (350, 450) for determining an anesthetic concentration in the region of an outlet (142) of the anesthetic dosing device (300, 400), comprising
- a first gas branch (110), which can be connected to a respiratory gas supply (112) and which is designed to guide a respiratory gas (104) through an anesthetic vaporizer (130) to a mixer unit (140),
- a second gas branch (120), which can be connected to the respiratory gas supply (112) and which is designed to likewise guide the respiratory gas (104) to the mixer unit (140),
- the anesthetic vaporizer (130), which is arranged and designed to enrich the respiratory gas (104) in the first gas branch (110) with an anesthetic (132),
- the mixer unit (140), which is arranged and designed to mix the respiratory gas (104) from the first gas branch (110), which is enriched with the anesthetic (132), with the respiratory gas (104) from the second gas branch (120), and to provide the resulting gas mixture (108) to the outlet (142) of the anesthetic dosing device (300, 400),
- the measuring unit (350, 450),
wherein the measuring unit (350, 450) comprises at least two sensor elements (352, 353),
wherein a first sensor element (352) of the at least two sensor elements (352, 353) is arranged and designed to measure at least one first characteristic value (154) of a gas concentration-dependent characteristic variable between the mixer unit (140) and the outlet (142), and
wherein a second sensor element (353) of the at least two sensor elements (352, 353) is arranged and designed to measure at least one second characteristic value (155) of the gas concentration-dependent characteristic variable in the second gas branch (120) or in the region of the respiratory gas supply (112),
wherein the measuring unit (350, 450) is further designed to determine, depending on calibration information associated with the second characteristic value (155) and at least the first characteristic value (154), the anesthetic concentration between the mixer unit (140) and the outlet (142) and to output a corresponding concentration signal (160),
wherein the measuring unit (350, 450) has at least two switches (358, 359),
wherein the first sensor element (352) of the at least two sensor elements (352, 353) is connected to a first switch (358) of the at least two switches (358, 359), and
wherein the second sensor element (353) of the at least two sensor elements (352, 353) is connected to a second switch (359) of the at least two switches (358, 359), and
wherein the first and the second switch (358, 359) are designed to be able to switch between the measurement of the first characteristic value (154) and the measurement of the second characteristic value (155) by the relevant connected sensor element (352, 353).

2. An anesthetic dosing device (300, 400) according to claim 1,
wherein the measuring unit (350, 450) is designed to determine the anesthetic concentration depending on the calibration information associated with the second characteristic value (155) and a difference between the first and the second characteristic value (154, 155)

3. An anesthetic dosing device (300, 400) according to at least one of the preceding claims,
wherein the at least two sensor elements (352, 353) measure the characteristic values (154, 155) of the gas concentration-dependent characteristic variable based on a thermal conductivity measurement or a density measurement.

4. An anesthetic dosing device (400) according to claim 3,
wherein the first switch (358) and the second switch (359) are coupled to one another in such a way that a change of a first switch position of the first switch (358) also causes a change of a second switch position of the second switch (359).

5. An anesthetic dosing device (300, 400) according to at least one of the preceding claims,
wherein the measurement of the first characteristic value (154) and the measurement of the second characteristic value (155) by the measuring unit (350) take place substantially simultaneously.

6. An anesthetic dosing device (300, 400) according to at least one of the preceding claims,
wherein the calibration information includes a plurality of respiratory gas composition-dependent calibration curves, in particular at least one corresponding respiratory gas composition-dependent calibration curve for an oxygen-nitrogen mixture and for an oxygen-nitrous oxide mixture.

7. An anesthetic dosing device (300, 400) according to at least one of the preceding claims,
wherein the measuring unit (350, 450) comprises a number of measuring gas guide channels (172, 174) in which the measurement of the first and the second characteristic value (154, 155) takes place, and which are pneumatically integrated into the respiratory gas guide of the anesthetic dosing device (300, 400) in such a way that temporary pressure fluctuations within the first and/or second gas branch (110, 120) are also at least partially present in a corresponding manner within the number of measuring gas guide channels (172, 174).

8. An anesthetic dosing device (400) according to at least one of the preceding claims,
wherein the anesthetic dosing device (400) furthermore comprises a control unit (490) which is designed to receive the output concentration signal (160) and, based on a comparison between a predetermined target anesthetic concentration and the determined anesthetic concentration, control the enrichment of the respiratory gas (104) with anesthetic (132) in the first gas branch (110).

9. A method (500) for determining an anesthetic concentration in the region of an outlet (142) of an anesthetic dosing device (300, 400) according to any of the preceding claims, comprising the steps of
- providing a first and a second gas branch (110, 120),
wherein the first gas branch (110) can be connected to a respiratory gas supply (112) and is designed to guide a respiratory gas (104) through an anesthetic vaporizer (130) to a mixer unit (140), and
wherein the second gas branch (120) can be connected to the respiratory gas supply (112) and is designed to likewise guide the respiratory gas (104) to the mixer unit (140), and
wherein the mixer unit (140) is arranged and designed to mix the respiratory gas (104) from the first gas branch (110), which is enriched with an anesthetic (132), with the respiratory gas (104) from the second gas branch (120), and to provide the resulting gas mixture (108) to an outlet (142) of the anesthetic dosing device (300, 400);
- measuring a first characteristic value (154) of a gas concentration-dependent characteristic variable between the mixer unit (140) and the outlet (142) by means of a first sensor element (352);
- measuring a second characteristic value (155) of the gas concentration-dependent characteristic variable in the second gas branch (120) or in the region of the respiratory gas supply (112) by means of a second sensor element (353);
- determining the anesthetic concentration between the mixer unit (140) and the outlet (142) based on calibration information associated with the second characteristic value (155) and at least the first characteristic value (154); and
- outputting a concentration signal (160) which indicates the determined anesthetic concentration, and
- switching between the measurement of the first characteristic value (154) and the measurement of the second characteristic value (155) by the relevant connected sensor element (352, 353).

10. The method (500) according to claim 9, further comprising the steps of
- receiving the output concentration signal (160);
- controlling the enrichment of the respiratory gas (104) with anesthetic (132) in the first gas branch (110) based on a comparison between a predetermined target anesthetic concentration and the determined anesthetic concentration.

## Revendications

1. Dispositif de dosage d'agent anesthésique (300, 400), comportant une unité de mesure (350, 450) permettant de déterminer une concentration en agent anesthésique dans la région d'une sortie (142) du dispositif de dosage d'agent anesthésique (300, 400), présentant
- une première branche à gaz (110) qui peut être reliée à une alimentation en gaz respiratoire (112) et qui est conçue pour guider un gaz respiratoire (104) vers une unité de mélangeur (140) à travers un évaporateur d'agent anesthésique (130),
- une seconde branche à gaz (120) qui peut être reliée à l'alimentation en gaz respiratoire (112) et qui est conçue pour guider le gaz respiratoire (104) également vers l'unité de mélangeur (140),
- l'évaporateur d'agent anesthésique (130), qui est disposé et conçu pour enrichir le gaz respiratoire (104) contenu dans la première branche à gaz (110) avec un agent anesthésique (132),
- l'unité de mélangeur (140), qui est disposée et conçue pour mélanger le gaz respiratoire (104) enrichi en agent anesthésique (132) et provenant de la première branche à gaz (110) au gaz respiratoire (104) provenant de la seconde branche à gaz (120) et pour fournir le mélange gazeux (108) qui en résulte à la sortie (142) du dispositif de dosage d'agent anesthésique (300, 400),
- l'unité de mesure (350, 450),
dans lequel l'unité de mesure (350, 450) présente au moins deux éléments capteurs (352, 353),
dans lequel un premier élément capteur (352) des au moins deux éléments capteurs (352, 353) est disposé et conçu pour mesurer, entre l'unité de mélangeur (140) et la sortie (142), au moins une première valeur caractéristique (154) d'une grandeur caractéristique dépendant de la concentration en gaz et
dans lequel un second élément capteur (353) des au moins deux éléments capteurs (352, 353) est disposé et conçu pour mesurer, dans la seconde branche à gaz (120) ou dans la région de l'alimentation en gaz respiratoire (112), au moins une seconde valeur caractéristique (155) de la grandeur caractéristique dépendant de la concentration en gaz,
dans lequel l'unité de mesure (350, 450) est en outre conçue pour déterminer, en fonction d'une information d'étalonnage associée à la seconde valeur caractéristique (155) et d'au moins la première valeur caractéristique (154), la concentration en agent anesthésique entre l'unité de mélangeur (140) et la sortie (142) et pour émettre un signal de concentration (160) correspondant,
dans lequel l'unité de mesure (350, 450) comprend au moins deux commutateurs (358, 359),
dans lequel le premier élément capteur (352) des au moins deux éléments capteurs (352, 353) est relié à un premier commutateur (358) des au moins deux commutateurs (358, 359) et
dans lequel le second élément capteur (353) des au moins deux éléments capteurs (352, 353) est relié à un second commutateur (359) des au moins deux commutateurs (358, 359) et
dans lequel les premier et second commutateurs (358, 359) sont conçus de façon à pouvoir passer, grâce à l'élément capteur (352, 353) relié à chacun d'eux, de la mesure de la première valeur caractéristique (154) à la mesure de la seconde valeur caractéristique (155).

2. Dispositif de dosage d'agent anesthésique (300, 400) selon la revendication 1,
dans lequel l'unité de mesure (350, 450) est conçue pour déterminer la concentration en agent anesthésique en fonction de l'information d'étalonnage associée à la seconde valeur caractéristique (155) et d'une différence entre la première et la seconde valeur caractéristique (154, 155).

3. Dispositif de dosage d'agent anesthésique (300, 400) selon au moins l'une des revendications précédentes,
dans lequel les au moins deux éléments capteurs (352, 353) mesurent les valeurs caractéristiques (154, 155) de la grandeur caractéristique dépendant de la concentration en gaz, sur la base d'une mesure de conductivité thermique ou d'une mesure de densité.

4. Dispositif de dosage d'agent anesthésique (400) selon la revendication 3,
dans lequel le premier commutateur (358) et le second commutateur (359) sont couplés entre eux de sorte qu'un changement d'une première position de commutation du premier commutateur (358) entraîne également un changement d'une seconde position de commutation du second commutateur (359).

5. Dispositif de dosage d'agent anesthésique (300, 400) selon au moins l'une des revendications précédentes,
dans lequel la mesure de la première valeur caractéristique (154) et la mesure de la seconde valeur caractéristique (155) se font sensiblement au même instant par l'unité de mesure (350).

6. Dispositif de dosage d'agent anesthésique (300, 400) selon au moins l'une des revendications précédentes,
dans lequel l'information d'étalonnage comprend une pluralité de courbes d'étalonnage dépendant de la composition du gaz respiratoire, en particulier au moins une courbe d'étalonnage respective, dépendant de la composition du gaz respiratoire, pour un mélange oxygène-azote et pour un mélange oxygène-gaz hilarant.

7. Dispositif de dosage d'agent anesthésique (300, 400) selon au moins l'une des revendications précédentes,
dans lequel l'unité de mesure (350, 450) présente un certain nombre de canaux de guidage de gaz de mesure (172, 174) dans lesquels la mesure de la première et de la seconde valeur caractéristique (154, 155) a lieu et qui sont intégrés pneumatiquement dans la conduite de gaz respiratoire du dispositif de dosage d'agent anesthésique (300, 400), de telle sorte que des fluctuations temporaires de pression à l'intérieur de la première et/ou de la seconde branche à gaz (110, 120) se présentent au moins partiellement de manière correspondante à l'intérieur du certain nombre de canaux de guidage de gaz de mesure (172, 174).

8. Dispositif de dosage d'agent anesthésique (400) selon au moins l'une des revendications précédentes,
dans lequel le dispositif de dosage d'agent anesthésique (400) présente en outre une unité de commande (490), qui est conçue pour recevoir le signal de concentration (160) émis et pour commander, sur la base d'une comparaison entre une concentration en agent anesthésique de consigne prédéterminée et la concentration en agent anesthésique déterminée, l'enrichissement du gaz respiratoire (104) en agent anesthésique (132) dans la première branche à gaz (110).

9. Procédé (500) de détermination d'une concentration en agent anesthésique dans la région d'une sortie (142) d'un dispositif de dosage d'agent anesthésique (300, 400) selon l'une des revendications précédentes, présentant les étapes de
- fourniture d'une première et d'une seconde branche à gaz (110, 120),
dans lequel la première branche à gaz (110) peut être reliée à une alimentation en gaz respiratoire (112) et est conçue pour guider un gaz respiratoire (104) vers une unité de mélangeur (140) à travers un évaporateur d'agent anesthésique (130) et
dans lequel la seconde branche à gaz (120) peut être reliée à l'alimentation en gaz respiratoire (112) et est conçue pour guider le gaz respiratoire (104) également vers l'unité de mélangeur (140) et
dans lequel l'unité de mélangeur (140) est disposée et conçue pour mélanger le gaz respiratoire (104) enrichi avec un agent anesthésique (132) provenant de la première branche à gaz (110) au gaz respiratoire (104) provenant de la seconde branche à gaz (120) et pour fournir le mélange gazeux (108) qui en résulte à une sortie (142) du dispositif de dosage d'agent anesthésique (300, 400) ;
- mesure, à l'aide d'un premier élément capteur (352), d'une première valeur caractéristique (154) d'une grandeur caractéristique, dépendant de la concentration en gaz, entre l'unité de mélangeur (140) et la sortie (142) ;
- mesure, à l'aide d'un second élément capteur (353), d'une seconde valeur caractéristique (155) de la grandeur caractéristique, dépendant de la concentration en gaz, dans la seconde branche à gaz (120) ou dans la région de l'alimentation en gaz respiratoire (112) ;
- détermination de la concentration en agent anesthésique entre l'unité de mélangeur (140) et la sortie (142), sur la base d'une information d'étalonnage associée à la seconde valeur caractéristique (155) et d'au moins la première valeur caractéristique (154) ; et
- émission d'un signal de concentration (160), lequel indique la concentration en agent anesthésique déterminée, et
- passage de la mesure de la première valeur caractéristique (154) à la mesure de la seconde valeur caractéristique (155) grâce à l'élément capteur (352, 353) respectivement relié.

10. Procédé (500) selon la revendication 9, présentant en outre les étapes de
- réception du signal de concentration (160) émis ;
- régulation de l'enrichissement du gaz respiratoire (104) en agent anesthésique (132) dans la première branche à gaz (110), sur la base d'une comparaison entre une concentration en agent anesthésique de consigne prédéterminée et la concentration déterminée en agent anesthésique.
